# EUROPEAN PATENT SPECIFICATION

(11) **EP 3 129 403 B1**
(45) Date of publication and mention of the grant of the patent: **07.11.2018**
(21) Application number: 15717533.2
(22) Date of filing: 01.04.2015
(51) Int. Cl.: C07K 16/14, G01N 33/577

(54) **ANTIBODY AGAINST HT-2 TOXIN-HT-2 TOXIN ANTIBODY COMPLEX**
ANTIKÖRPER GEGEN HT-2-TOXIN-HT-2-TOXIN-ANTIKÖRPERKOMPLEX
ANTICORPS CONTRE LE COMPLEXE TOXINE HT-2-ANTICORPS ANTI-TOXINE HT-2

(30) Priority: 07.04.2014 FI 20145330
(43) Date of publication of application: 15.02.2017
(73) Proprietor: Teknologian Tutkimuskeskus VTT OY, 02150 Espoo (FI)
(72) Inventor: AROLA, Henri, FI-02044 VTT (FI); TULLILA, Antti, FI-02044 VTT (FI); NEVANEN, Tarja, FI-02044 VTT (FI)
(74) Representative: Boco IP Oy Ab
(86) International application number: PCT/FI2015/050230
(87) International publication number: WO 2015/155412

(56) References cited:
- CN-A- 102 766 208
- YANSHEN LI ET AL: "High Specific Monoclonal Antibody Production and Development of an ELISA Method for Monitoring T-2 Toxin in Rice", JOURNAL OF AGRICULTURAL AND FOOD CHEMISTRY, vol. 62, no. 7, 19 February 2014 (2014-02-19), pages 1492-1497, XP055194448, ISSN: 0021-8561, DOI: 10.1021/jf404818r
- TURNER N W ET AL: "Analytical methods for determination of mycotoxins: A review", ANALYTICA CHIMICA ACTA, ELSEVIER, AMSTERDAM, NL, vol. 632, no. 2, 26 January 2009 (2009-01-26), pages 168-180, XP025817243, ISSN: 0003-2670, DOI: 10.1016/J.ACA.2008.11.010 [retrieved on 2008-12-22]

## Description

### FIELD OF THE INVENTION

The present invention relates to immunodiagnostics. Especially, the invention relates to an enhanced immunoassay for detecting the presence of HT-2 toxin. In particular the invention relates to a binding partner capable of recognizing an immune complex between HT-2 toxin and anti-HT-2 toxin antibody. The invention also relates to a test kit comprising said binding partner for detecting HT-2 toxin. Further, the invention relates to a method and an immunoassay, wherein said binding partner is used. Furthermore, the invention relates to a polynucleotide encoding said binding partner and a host cell capable of expressing said binding partner.

### BACKGROUND

Mycotoxins are toxic secondary metabolites of fungi and one of the major contaminants of agricultural products. FAO has estimated that mycotoxins affect one quarter of world's food crops. All the factors inducing mycotoxin production are not known and therefore the production is not predictable.

Mycotoxins are small organic molecules resistant to various methods of ravaging. The contamination of mycotoxins in food, feed and raw materials (such as wheat, barley, rye and maize) is an increasing problem worldwide. Mycotoxins are also harmful for the microbes used in food processes. More than 300 mycotoxins are known with differing toxic modes of action to humans and animals.

Mycotoxins contaminate food and feed from field to consumer products. Huge lots of raw material has to be sampled before accepted to further use. Mycotoxins also cause illnesses in human and animals eating the mycotoxin contaminated food and feed. HT-2 and T-2 toxins are secondary metabolites of *Fusarium spp* fungi which contaminates cereals already in the fields. These toxins inhibit both DNA and protein synthesis. HT-2 trichothecenes can cause e.g. hemorrhage, vomitting, impaired immune function, fever and headache.

For example European Union has established recommendations of maximum permissible limits to circa ten mycotoxins or derivatives thereof in raw materials and certain products. Sampling, sample preparation and analysis is of critical importance since failure to achieve a satisfactory verified analysis can lead to unacceptable consignments being accepted or satisfactory loads being unnecessarily rejected. Advances in chromatographic methods, liquid and gas chromatography, integrated to mass spectrometry have been reported (Li et al. 2013 Review). These methods are sensitive and accurate although tedious sample preparation is needed. In addition expensive equipment and highly skilled personnel working in laboratory environment are required.

Fast and simple *on-site* methods suitable for high-throughput assay format are needed. The current immunoassays for determining HT-2 toxin are not satisfactory. They are based on antibodies and are competitive immunoassays which are sensitive to the cross-reacting molecules in a sample. When a competitive assay is used the presence of cross-reactive molecules in the sample gives overestimation of the actual analyte. Quantitative competitive ELISA assays require a complex multistep procedure with several washing steps.

In open sandwich immunoassay only one antibody is needed and the signal amplification is obtained via conjugated enzyme (Ueda et al. 1996, Hara et al. 2013). Coating, blocking, several incubation and washing steps, however, take as much time as the conventional sandwich ELISAs. Assays based on a single antibody are sensitive to other related analytes due to the cross-reactivity.

Phage immune complex assay reported by Gonzales-Techera et al. (2007) has the potential for the ELISA type of assay or PCR amplification of the signal. Phages as such are not preferred detection bodies in fast diagnostics and *in situ* use.

The quenching of the intrinsic fluorescence of antibodies due to the binding of a small analyte has been utilized in a non-competetive assay development for mycotoxins aflatoxin, ochratoxin A and zearalone by Li et al. (2013). Method uses one antibody which specificity determines the specificity of the assay. Binding of cross-reacting molecules may induce the fluorescent quenching as well as the target analyte. Another approach utilizes the FRET phenomenon between antibody and bound small analyte (Li et al. 2011). This method is applicable for limited amount of organic molecules as a target analyte.

Simple lateral flow tests are widely used in various application areas due to their ease of use and fast response but they allow only qualitative or semi-quantitative result (Dzantiev et al. 2014 review). Vanrell et al. (2013) have developed further a small analyte lateral flow test utilizing anti-immune complex peptides inserted to the streptavidin/avidin core.

EP 1579222B1 discloses a non-competitive immunoassay for assaying small analytes. CN 102766208A discloses a monoclonal antibody capable of identifying T-2 and HT-2 toxin. Also an enzyme-linked immunosorbent assay method and a kit for detecting T-2 and HT-2 toxin, and application thereof to detection of HT-2 and T-2 toxin, are disclosed.

Yanshen et al. (2014) discloses the creation of a specific anti-T-2 toxin monoclonal antibody (mAb) which binds to T-2 toxin with negligible CR value to most mycotoxins, including HT-2 toxin. Using this specific antibody, an ELISA method for the determination of T-2 toxin is disclosed.

Turner et al. (2009) discloses mycotoxins, such as Ochratoxins and Aflatoxins. Turner et al. describes that due to the variety of structures of these toxins, it is impossible to use one standard technique for analysis and/or detection and that practical requirements for high-sensitivity analysis and the need for a specialist laboratory setting create challenges for routine analysis. Turner et al. also discloses several analytical techniques, which offer flexible and broad-based methods of analysis and in some cases detection.

Thus there is still a need for an easy, rapid and reliable test for testing HT-2 toxin, which may be used e.g. for screening large amounts of samples.

### SUMMARY OF THE INVENTION

The present invention provides novel approaches for fast diagnostics for the screening of the presence of mycotoxins in high-throughput manner from abundant sample amounts.

The present invention relates to a novel reagent for rapid and reliable detection of HT-2 mycotoxin. The reagent is capable of recognizing an immune complex formed between HT-2 toxin and anti-HT-2-toxin antibody. Especially, the present invention relates to a binding partner comprising the complementary determining regions (CDRs) of an antibody light chain and heavy chain, wherein said light chain regions have the amino acid sequences of amino acids 24-37, 53-59 and 92-102 of SEQ ID NO:7, respectively, and said heavy chain regions have the amino acid sequences of amino acids 31-35, 50-65 and 99-107 of SEQ ID NO:8, respectively.

The invention also relates to a test-kit comprising said binding partner and further comprising an another binding partner comprising light chain CDRs1-3 comprising amino acids 24-34, 50-56, and 89-97 of SEQ ID NO:3, respectively, and heavy chain CDRs1-3 comprising amino acids 31-36, 51-66, and 99-105 of SEQ ID NO:4, respectively.

The invention also relates to the use of the binding partners for detecting HT-2 toxin in a sample. The binding partners especially suitable for a non-competitive homogenous immunoassay that may be used e.g. for testing large amount of samples.

Furthermore, the invention provides a method for detecting an analyte in a sample, whereby the sample is reacted with a reagent pair comprising a first binding partner that specifically binds to the analyte, and a second binding partner that specifically binds to the complex of the analyte and the first binding partner, and determining the binding of the second binding partner to said complex, thus indicating the presence of the analyte in the sample, wherein said analyte is a HT-2 toxin, and said first binding partner comprises light chain CDRs1-3 comprising amino acids 24-34, 50-56, and 89-97 of SEQ ID NO:3, respectively, and heavy chain CDRs1-3 comprising amino acids 31-36, 51-66, and 99-105 of SEQ ID NO:4, and said second binding partner comprises light chain CDRs1-3 comprising amino acids 24-37, 53-59, and 92-102 of SEQ ID NO:7, respectively, and heavy chain CDRs1-3 comprising amino acids 31-35, 50-65, and 99-107 of SEQ ID NO:8, respectively. Yet in addition the invention provides a polynucleotide encoding the binding partner, and a host cell capable of expressing the binding partner. The polynucleotide is DNA.

In addition the invention provides use of the binding partner for detecting an analyte in a food or animal feed sample, in a sample taken from a human or an animal exposed to a HT-2 toxin, or in an environmental sample.

The present description discloses a simple one-step immunoassay for HT-2 toxin.

Advantageous embodiments of the invention are set forth in the dependent claims. Other objects, details and advantages of the invention will become apparent from the following drawings, detailed description and examples.

### BRIEF DESCRIPTION OF THE DRAWINGS

**Figure 1a** shows the nucleotide sequence of the HT2-10 Fab light chain (SEQ ID NO:1).
**Figure 1b** shows the nucleotide sequence of the HT2-10 Fab heavy chain (SEQ ID NO:2).
**Figure 2a** shows the amino acid sequence of the HT2-10 Fab light chain (SEQ ID NO:3). CDR regions are underlined and marked in bold. The three complementary regions LCDR1 (amino acids 24-34, according to Kabat 24-34), LCDR2 (amino acids 50-56, according to Kabat 50-56) and LCDR3 (amino acids 89-97, according to Kabat 89-97) of each immunoglobulin chain are shown.
**Figure 2b** shows the amino acid sequence of the HT2-10 Fab heavy chain (SEQ ID NO: 4). CDR regions are underlined and marked in bold. The three complementary regions HCDR1 (amino acids 31-36, according to Kabat 31-35), HCDR2 (amino acids 51-66, according to Kabat 50-65) and HCDR3 (amino acids 99-105, according to Kabat 95-102) of each immunoglobulin chain are shown.
**Figure 3a** shows the nucleotide sequence of the Anti-IC HT2-10 (H5) light chain (SEQ ID NO: 5).
**Figure 3b** shows the nucleotide sequence of the Anti-IC HT2-10 (H5) heavy chain (SEQ ID NO: 6).
**Figure 4a** shows the amino acid sequence of the Anti-IC HT2-10 (H5) light chain (SEQ ID NO: 7). CDR regions are underlined and marked in bold. The three complementary regions LCDR1 (amino acids 24-37, according to Kabat 24-34), LCDR2 (amino acids 53-59, according to Kabat 50-56) and LCDR3 (amino acids 92-102, according to Kabat 89-97) of each immunoglobulin chain are shown.
**Figure 4b** shows the amino acid sequence of the Anti-IC HT2-10 (H5) heavy chain (SEQ ID NO: 8). CDR regions are underlined and marked in bold. The three complementary regions HCDR1 (amino acids 31-35, according to Kabat 31-35), HCDR2 (amino acids 50-65, according to Kabat 50-65) and HCDR3 (amino acids 99-107, according to Kabat 95-102) of each immunoglobulin chain are shown.

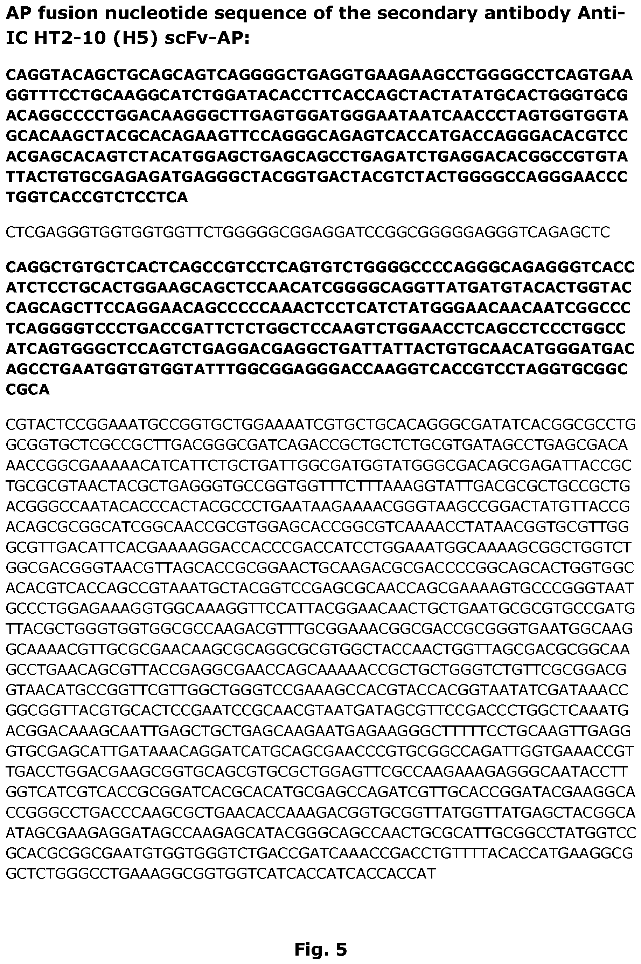

**Figure 5** shows the nucleotide sequence of the AP fusion of the secondary antibody Anti-IC HT2-10 (H5) scFv-AP. Heavy and light variable regions are marked in bold.
Heavy variable and light variable region are connected with a linker. Alkaline phosphatase is at the end of the antibody fragment.

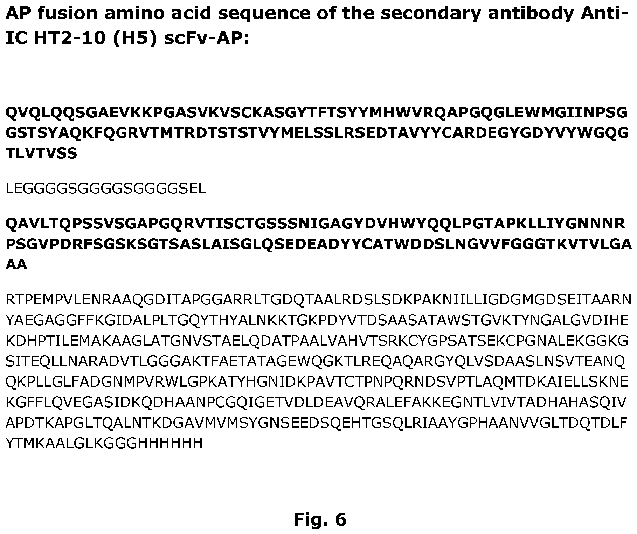

**Figure 6** shows the amino acid sequence of AP fusion of the secondary antibody Anti-IC HT2-10 (H5) scFv-AP. Heavy and light variable regions are marked in bold. Heavy variable and light variable region are connected with a linker. Alkaline phosphatase is at the end of the antibody fragment, followed by a histidine tag.
**Figure 7** shows HT-2 toxin assay specificity. The test results of HT-2 toxin FRET assay in PBS, T-2 toxin background, 5 min assay time are provided. Anti- immune complex HT2-10 (H5) was selected from VTT human naïve library. The primary antibody HT-2 10 has cross-reactivity of 100% for HT2 and T-2 toxin. The secondary anti-IC antibody makes the assay specific for HT-2.
**Figure 8** shows HT-2 toxin assay specificity in simple ELISA assay in PBS, 50 min, T-2 toxin background, Average of 3 replicates.
**Figure 9** shows HT-2 toxin FRET assay in PBS, 10 min assay time, Average of 6 replicates. A standard curve is presented.
**Figure 10** shows the test results of HT-2 toxin FRET assay in 10 % methanol/water, Average of 6 replicates, 10 min assay time. A standard curve is presented. A situation where the HT-2 toxin is in 70% MeOH/water and diluted 1:7.
**Figure 11** shows the test results of HT-2 toxin Fret Assay. The reaction was carried out in PBS with dried antibody reagents. TR-FRET was measured after 10 min. Average of 6 replicates.
**Figure 12** shows HT-2 toxin FRET assay 10 min in 10 % methanol/water, Dried antibody reagents, Average of 6 replicates.
**Figure 13** shows the test results of HT-2-toxin Simple ELISA standard curve in PBS with average of 6 replicates, 60 min.
**Figure 14** shows Simple ELISA assay in 10 % MeOH, 60 min, 6 replicates.
**Figure 15** shows the lateral flow assay for HT-2 toxin where sample without toxin doesn't give background (left), clear band is obtained with HT-2 toxin sample (middle) and no band with T-2 toxin sample is seen (right).

### SEQUENCE LISTING

### Primary antibody HT2-10 sequences

SEQ ID NO:1: HT2-10 Fab Light Chain nucleotide sequence
SEQ ID NO:2: HT2-10 Fab Heavy Chain nucleotide sequence
SEQ ID NO:3: HT2-10 Fab Light Chain amino acid sequence
SEQ ID NO:4: HT2-10 Fab Heavy chain amino acid sequence

### Secondary antibody Anti-IC HT2-10 (H5) sequences

SEQ ID NO:5: Anti-IC HT2-10 (H5) Light Chain nucleotide sequence
SEQ ID NO:6: Anti-IC HT2-10 (H5) Heavy Chain nucleotide sequence
SEQ ID NO:7: Anti-IC HT2-10 (H5) Light Chain amino acid sequence
SEQ ID NO:8: Anti-IC HT2-10 (H5) Heavy Chain amino acid sequence
SEQ ID NO:9: AP fusion nucleotide sequence of the secondary antibody Anti-IC HT2-10 (H5) scFv-AP
SEQ ID NO: 10: AP fusion amino acid sequence of the secondary antibody Anti-IC HT2-10 (H5) scFv-AP

### DETAILED DESCRIPTION OF THE INVENTION

HT-2 and T-2 are mycotoxins, which are secondary metabolites of *Fusarium spp* fungi. HT-2 is a hydrolyzed form of T-2 toxin. HT-2 and T-2 toxins occur in oat, barley, wheat, rye, maize, rice, and products made thereof such as noodles, beer and cereal-based baby food.

HT-2 and T-2 mycotoxins belong to trichothecene mycotoxins, which have several health related issues: trichothecenes cause hemorrhage, vomitting, impaired immune function, fever, headache and even death.

The present invention relates to a fast and simple assay for mycotoxinanalysis. Especially the present invention relates to an immunoassay for HT-2 toxin detection. One-step and reliable rapid analysis is suitable for small analytes. Sandwich type assay for small analytes provides improved specificity and sensitivity. Development of anti-immune complex antibodies is carried out.

An advantage of the present invention is an improvement the specificity of the HT-2 toxin immunoassay and significant simplification of the assay procedure compared to the currents assays. This reduces the amount of work and thus the time needed.

The immunoassay of the present invention is fast and can be applied to high through-put assays for large number of samples. The present invention improves the specificity of the assay.

The reagent pair for the non-competitive immunoassay of the invention comprises a first binding partner and a second binding partner. The first binding partner binds to the analyte to form a complex between the first binding partner and the analyte. The second binding partner binds to the complex formed by the first binding partner and the analyte. The binding partners are usually proteins such as antibodies including antibody fragments that have the desired binding properties.

"Binding partner" as used herein is usually a protein or a polypeptide, such as antibodies including antibody fragments that have the desired binding properties. An antibody is an immunoglobulin molecule and it can belong to any of classes IgG, IgIvI, IgE, IgA or IgD; IgG and IgM being the most frequently used. Preferably the binding partners are antibody fragments comprising the ligand-binding site, such as Fab, or scFv fragments. The fragment known as the Fab fragment (fragment antigen-binding) consists of the variable and constant domain of an immunoglobulin light chain covalently attached by a disulfide bridge to the variable and first constant domain of an immunoglobulin heavy chain. Fv (variable domain) means the variable regions of the immunoglobulin molecule that are responsible for the ligand binding. ScFv (single chain Fv) means a molecule wherein the variable domains of the heavy and light chain of an antibody are linked by a peptide to form a single polypeptide chain synthesized from a single mRNA molecule. The variable regions of an immunoglobulin heavy chain and light chain are together responsible for the ligand binding. Ligand is the substance to which the binding partner binds, in connection with antibodies it is an antigen or a hapten.

The first binding partner of the present invention comprises light chain CDRs1-3 comprising amino acids 24-34, 50-56, and 89-97 of SEQ ID NO:3, respectively, and heavy chain CDRs1-3 comprising amino acids 31-36, 51-66, and 99-105 of SEQ ID NO:4. The first binding partner anti-HT2 Fab (10) is also disclosed in the present description by such names as primary antibody HT2-10, anti-HT-2 toxin antibody, primary antibody HT2-10, HT2-10 Fab, anti-HT2 toxin Fab, primary antibody HT2-10 Fab and anti- HT2/T2 Fab(10).

The second binding partner of the present invention comprises light chain CDRs1-3 comprising amino acids 24-37, 53-59, and 92-102 of SEQ ID NO:7, respectively, and heavy chain CDRs1-3 comprising amino acids 31-35, 50-65, and 99-107 of SEQ ID NO:8, respectively. The second binding partner anti-IC-HT2 Fab (H5) is also disclosed in the present description by such names as anti-IC HT2-10 (H5), secondary antibody anti-IC HT2-10 (H5), anti- immune complex HT2-10 (H5), secondary anti-IC antibody, anti-IC-HT2-10(H5), secondary antibody H5, anti- immune complex antibody H5 and anti-IC-HT2 (H5) Fab.

As used herein, the term "hypervariable region" refers to the amino acid residues of an antibody which are responsible for antigen binding. The hypervariable region comprises amino acid residues from a "Complementarity Determining Region" or "CDR" (*e*.*g*.,. residues 24-34 (L1), 50-56 (L2) and 89-97 (L3) in the light chain variable domain and 31-35 (H1), 50-65 (H2) and 95-102 (H3) in the heavy chain variable domain according to Kabat et al., Sequences of Proteins of Immunological Interest, 5th Ed. Public Health Service, National Institutes of Health, Bethesda, Md. (1991)) and/or those residues from a "hypervariable loop". "Framework Region" or "FR" residues are those variable domain residues other than the hypervariable region residues as herein defined. As used herein, the terms "heavy chain variable domain," "V_{H} domain" and/or "V_{H}" are used interchangeably and reference the hypervariable region (encompassing both the CDR and framework domains) of the heavy chain of an antibody; the terms "light chain variable domain," "V_{L} domain" and/or "V_{L}" are used interchangeably and reference the hypervariable region (encompassing both the CDR and framework domains) of the heavy chain of an antibody.

In the present invention the first binding partner i.e. the primary antibody HT2-10 comprises light chain CDRs1-3 comprising amino acids 24-34, 50-56, and 89-97 of SEQ ID NO:3, respectively, and heavy chain CDRs1-3 comprising amino acids 31-36, 51-66, and 99-105 of SEQ ID NO:4, respectively.

In the present invention the second binding partner i.e. the secondary antibody Anti-IC HT2-10 comprises light chain CDRs1-3 comprising amino acids 24-37, 53-59, and 92-102 of SEQ ID NO:7, respectively, and heavy chain CDRs1-3 comprising amino acids 31-35, 50-65, and 99-107 of SEQ ID NO:8, respectively.

The first and second binding partners can form antibody fragments Fab, scFv, and recombinant monoclonal antibodies.

The recombinant binding partner library is conveniently an expression library, which is typically a display library. The general principle of the display recombinant binding partner libraries is that they present the binding partner as a fusion protein on the surface, which may be the surface of a microbial cell such as a yeast or bacterial cell, or a bacteriophage. The display recombinant binding partner library can also be a display library, where stable complexes of nascent protein and mRNA are produced in an in vitro expression system. Phage display libraries are the most frequently used.

The first binding partner may be a conventional monoclonal antibody or fragment thereof, but preferably it is a recombinant one, as is the second binding partner.

The binding partner of the invention may be used in a sandwich assay for detecting HT-2 toxin in a sample, whereby the sample is reacted with a reagent pair comprising a first binding partner that specifically binds to HT-2 toxin and a second binding partner, which is capable of specifically recognizing an immune complex between HT-2 toxin and anti-HT-2 toxin antibody, and which is the binding partner of the invention. Binding of the second binding partner to the immune complex between the first binding partner and HT-2 toxin indicates the presence of HT-2 toxin in the sample.

The binding partners are conveniently prepared using a recombinant phage display binding partner library e.g. as described in WO 2004/046733. When the first binding partner has been selected for, it is complexed with its ligand and this complex is used to select for the second binding partner from a recombinant antibody library. The first binding partner without the ligand is used as contraselection. The second binding partner should only recognize complexes, not free first binding partner nor free antigen to any significant extent.

A phage display antibody library may be constructed by cloning immunoglobulin domains coding cDNAs into an appropriate phage display vector.

DNA encoding for millions of variants of antibody fragments is batch-cloned into the vector as part of the phage coat protein. Large libraries containing millions of antibody fragments with different specificities can be obtained by transforming the vectors in bacteria. Cultivation of the bacteria leads to the expression of phages displaying antibody fragments on their surface. The gene for the displayed antibody is carried in the phagemid plasmid packed inside the phage, thus linking genotype with phenotype. The physical linkage between the displayed protein and its DNA allows screening of vast numbers of variants of the protein, each linked to its corresponding DNA, by a simple *in vitro* selection procedure called panning.

In its simplest form, panning is carried out by incubating the pool of phage displayed variants with the ligand of interest that has been immobilized on a carrier, washing away unbound phage, and eluting specifically bound phage by disrupting the binding to the ligand. The eluted phage is then amplified *in vivo.*

The process is repeated several times, resulting in stepwise enrichment of the phage pool in favour of the tightest binding sequences. After about 3 to 6 rounds of selection and amplification, the best clones are sequenced and transformed into a host cell for further expression. The host cell may be a eukaryotic or prokaryotic cell e.g. a yeast, animal, plant or insect cell or bacterial cell. It may even be a mammalian cell, which after transformation produces a recombinant monoclonal antibody. The recombinant binding partner or at least part of it may also be produced synthetically.

The present invention relates to novel mycotoxin specific binding partners. More particularly the invention relates to mycotoxin specific recombinant antibodies. Benefits of the recombinant antibodies include reduced size, efficient immobilization, and cheap bacterial production. Antibody libraries enable fast *in vitro* selection of novel antibodies. The use of challenging antigens is accomplished. Antibody libraries contain ∼10⁸ different clones.

Selection of recombinant antibody libraries can be carried out e.g. by automated magnetic bead processor. Several different selection methods can be used to select specific antibodies. Single recombinant antibody clones are screened e.g. by a high throughput robotic station. Thousands of clones can be screened per day. Preliminary characterization of the candidate antibodies is carried e.g. by ELISA and sequence comparison.

A subset of strains containing antibodies has been deposited according to the Budapest Treaty on the International Recognition of the Deposit of Microorganisms for the Purposes of Patent Procedure, 1977, in VTT Culture Collection, P.O. Box 1000, FI-02044 VTT, FINLAND (http://culturecollection.vtt.fi), with the following codes (E-number):
HT2-10 Fab/pKKtac/RV308; E-143342
Anti-IC HT-10 (H5) Fab/pjExpress401/RV308; E-143343

The reagent pair comprising a first binding partner recognizing HT-2 and T-2 toxin, and a second binding partner recognizing an immune complex between HT-2 toxin and said first binding partner makes a non-competitive sandwich assay for HT-2 toxin feasible. The sandwich can be detected by all the standard immunoassay methods. One partner may be immobilized on a carrier, such as a microtiter well, µ-fluidic system, strip or a bead. A sandwich is formed in the presence of analyte and the other binding partner. The sandwich may be detected e.g. by using secondary antibodies or by labeling at least one of the binding partners.

The label can be any conventional label, such as a radioactive label, an enzyme, a nucleotide sequence or a fluorescent compound. The assay can be e.g. ELISA, immunoPCR or FIA.

Detection mode can be colorimetric, fluorescent (either FRET or surface fluorescence), paramagnetic, electrochemical or label free (e.g surface plasmon resonance and quartz crystal microbalance) Also other assay types, such as agglutination test, lateral flow test, capillary electrophoresis, antibody arrays, cantilevers and microfluidic assay systems, can be applied.

A great advantage of a reagent pair comprising the binding partner of the present invention is that it enables a homogenous immunoassay, i.e. an immunoassay that is carried out in solution. The avoidance of immobilizing and washing steps makes the assay extremely simple. The homogenous assay provides an excellent and convenient tool for on-site tests e.g. to be used by the customs to inspect exported raw materials, goods etc. The sample to be analyzed may be any food sample, feed sample, food raw material sample, feed raw material sample, or a sample originating from a human or an animal which has been consuming HT-2 toxin contaminated food or feed. The sample may originate e.g. from oat, barley, wheat, rye, maize, rice, and products made thereof such as noodles, beer and cereal based baby food.

A preferred homogenous immunoassay is one based on fluorescence resonance energy transfer (FRET). In FRET, energy from a molecular fluorophore (donor) is excited to a high-energy state and transferred to another fluorophore (acceptor) via intermolecular dipole-dipole coupling. This is possible only if the distance between the donor and the acceptor is short (1-10 nm) and the fluorescence spectrum of the donor and the absorption spectrum of the acceptor partially overlap. The energy transfer is then detected as a change in fluorescence. Often time-resolved fluorescence is utilized (Hemmilä I. et al., 1988, Clin. Chem. 34:2320-2322).

FRET may be applied to the present invention by labeling the two binding partners, which preferably are antibody fragments, with fluorophores that form a FRET donor-acceptor pair. When the binding partners and the analyte are small the fluorophores come into very close proximity, and a measurable FRET signal is obtained.

Alternatively the immunoassay for HT-2 toxin use may be for example a conventional sandwich test in microtiter wells or a lateral flow-test.

The binding partner of the invention may be included in a test-kit, which may further contain any other reagents needed for the assay together with instructions for use. Preferably the test-kit contains also a first binding partner for binding the analyte. More preferably said binding partner comprises the complementary determining regions (CDRs) of an antibody light chain and heavy chain, wherein said light chain regions have the amino acid sequences of amino acids 24-34, 50-56, and 89-97 of SEQ ID NO:3, and said heavy chain regions have the amino acid sequences of amino acids 31-36, 51-66, and 99-105 of SEQ ID NO:4. Preferably the test-kit comprises reagents for carrying out a non-competitive, homogenous assay such as e.g. reagents needed for a TR-FRET test. The kit may further comprise reaction solutions, buffers, washing solutions and detecting means, such as labels and optionally a fluorometer.

The performance of the test may be such that the reagents are in the well of a microtiter plate and dilution series of sample is added. In a preferred mode for e.g. field use, the reagents are in dry form in a vessel.

The sample is added, which dissolves the reagents and the result can be read without further processes.

In that case the test-kit may comprise multiple reagent pairs physically separated from each other e.g. in the form of a microarray, whereby multiple analytes may be tested simultaneously from a sample. The present invention can be applied to high through-put assays for large number of samples.

The binding partner of the invention comprises three CDRs of an antibody light chain having the sequences as set forth in SEQ ID NO:7, and three CDRs of an antibody heavy chain having the sequences as set forth in SEQ ID NO:8. According to one embodiment of the invention the binding partner comprises the whole variable region i.e. the ligand-binding portion of said light chain (VL) and heavy chain (VH). It may thus contain amino acids 1-107 of SEQ ID NO:7 and amino acids 1-118 of SEQ ID NO:8. In particular said binding partner comprises the amino acid sequences of SEQ ID NO:7 and NO:8. Correspondingly the first binding partner may comprise the whole variable region of SEQ ID NO:3, and of SEQ ID NO:4, i.e. amino acids 1-107 of SEQ ID NO:3 and amino acids 1-116 of SEQ ID NO:4. Especially it comprises the amino acid sequences of SEQ ID NO:3 and NO:4.

The development of immune complex antibodies is difficult. The present invention provides for the first time an immune complex antibody for HT-2. The second binding partner comprising complementary determining regions (CDRs) with light chain regions having the amino acids 24-37, 53-59 and 92-102 of SEQ ID NO:7, and heavy chain regions having the amino acid sequences of amino acids 31-35, 50-65, and 99-107 of SEQ ID NO:8 of the present invention is specific for HT-2. The first binding partner comprising complementary determining regions (CDRs) having the amino acid sequences of amino acids 24-34, 50-56, and 89-97 of SEQ ID NO:3, and said heavy chain regions have the amino acid sequences of amino acids 31-36, 51-66, and 99-105- of SEQ ID NO:4.

The present invention can be applied in the fast diagnostics of HT-2 toxin in food, feed and food and feed related materials. Other application areas include e.g. diagnostics of human and animal exposure to the HT-2 toxin, or use as a research tool.

According to the EU recommendation 2013/165/EU:
In the case of the use of an analytical screening technique, the limit of detection should preferably not be higher than 25 µg/kg for the sum of T-2 and HT-2 toxin, more preferably not higher than 24, 23, 22, 21, 20, 19, 18, 17, 16, 15, 14, 13, 12, 11, 10, 9, 8, 7, 6, 5, 4, 3, 2, or 1 µg/kg for the sum of T-2 and HT-2 toxin.

The use of raw materials requires pre-treatment steps and the treatments may differ depending on the material.

The antibodies of the present invention can be used in sensors based on immunoassays. A skilled person in the art is able to choose suitable sensors to be used.

Minor variations or modifications of any one of the sequences or subsequences set forth in the description and claims are still within the scope of the invention provided that they do not affect the binding activity of the proteins.
In embodiment 1 the present invention provides a binding partner comprising light chain CDRs1-3 comprising amino acids 24-37, 53-59, and 92-102 of SEQ ID NO:7, respectively, and heavy chain CDRs1-3 comprising amino acids 31-35, 50-65, and 99-107 of SEQ ID NO:8, respectively.
In embodiment 2 is provided the binding partner according to embodiment 1, wherein the binding partner is an antibody or an antibody fragment.
In embodiment 3 is provided the binding partner according to embodiment 1 or 2, wherein the binding partner forms an antibody fragment Fab, scFv, or a recombinant monoclonal antibody.
   The binding partner according to embodiment 1 or 2 may comprise a purified antibody comprising light chain CDRs 1-3 and heavy chain CDRs1-3.
In embodiment 4 is provided the binding partner according to any one of embodiments 1 or 3, wherein the binding partner comprises amino acid sequences of SEQ ID NO:7 and SEQ ID NO:8.
   According to an embodiment of the description the binding partner comprises the whole variable region i.e. the ligand-binding portion of said light chain (VL) and heavy chain (VH). It may thus contain amino acids 1-107 of SEQ ID NO:7 and amino acids 1-118 of SEQ ID NO:8. In particular said binding partner comprises the amino acid sequences of SEQ ID NO:7 and NO:8.
In embodiment 5 is provided the binding partner according to any one of embodiments 1 to 4, which specifically recognizes an immune complex between HT-2 toxin and anti-HT-2 toxin.
In embodiment 6 is provided a test-kit comprising the binding partner according to any one of embodiments 1 to 5.
In embodiment 7 is provided the test-kit according to embodiment 6, further comprising another binding partner comprising light chain CDRs1-3 comprising amino acids 24-34, 50-56, and 89-97 of SEQ ID NO:3, respectively, and heavy chain CDRs1-3 comprising amino acids 31-36, 51-66, and 99-105 of SEQ ID NO:4, respectively.
In embodiment 8 is provided the test kit according to embodiment 7, wherein said another binding partner is an antibody or an antibody fragment.
   In an embodiment another binding partner may comprise the whole variable region of SEQ ID NO:3, and of SEQ ID NO:4, i.e. amino acids 1-107 of SEQ ID NO:3 and amino acids 1-116 of SEQ ID NO:4. Especially it comprises the amino acid sequences of SEQ ID NO:3 and NO:4.
In embodiment 9 is provided the test kit according to embodiment 7 to 8, wherein said another binding partner forms an antibody fragment Fab, scFv, or a recombinant monoclonal antibody.
In embodiment 10 is provided the test-kit according to any one of embodiments 7 to 9, wherein said another binding partner specifically recognizes HT-2 toxin, or both HT-2 toxin and T-2 toxin.
In embodiment 11 is provided the test kit according to any one of embodiments 7 to 10, comprising reagents for a non-competitive, homogenous immunoassay.
In embodiment 12 is provided the test kit according to any one of embodiments 7 to 11, comprising reagents for time resolved fluorescent resonance energy transfer (TR-FRET) assay.
In embodiment 13 is provided a polynucleotide encoding the binding partner according to any one of embodiments 1 to 5.
   A polynucleotide may be DNA comprising cDNA encoding a polypeptide comprising the binding partner according to any one of embodiments 1 to 5.
In embodiment 14 is provided a polynucleotide encoding said another binding partner referred to in any one of embodiments 7 to 12.
In embodiment 15 is provided an isolated host cell that has been transformed with a polynucleotide encoding the binding partner according to any one of embodiments 1 to 5 and being capable of expressing the binding partner according to any one of embodiments 1 to 5.
In embodiment 16 is provided an isolated host cell that has been transformed with a polynucleotide according to embodiment 15.
In embodiment 17 is provided use of the binding partner according to any one of embodiments 1 to 5 for detecting an analyte in a food or animal feed sample.
In embodiment 18 is provided use of the binding partner according to any one of embodiments 1 to 5 for detecting an analyte in a sample taken from a human or an animal exposed to a HT-2 toxin.
In embodiment 19 is provided use of the binding partner according to any one of claims 1 to 5 for detecting an analyte in an environmental sample.
In embodiment 20 is provided a method for detecting HT-2 toxin in a sample, comprising
   - reacting the sample with a reagent pair comprising a first binding partner that binds to a HT-2 toxin or T-2 toxin in the sample to form a complex, and a second binding partner that specifically binds to said complex; and
   - determining the binding of the second binding partner to the complex, thereby indicating presence of the HT-2 toxin in the sample,
   wherein said first binding partner comprises light chain CDRs1-3 comprising amino acids 24-34, 50-56, and 89-97 of SEQ ID NO:3, respectively, and heavy chain CDRs1-3 comprising amino acids 31-36, 51-66, and 99-105 of SEQ ID NO:4, respectively, and
   said second binding partner comprises light chain CDRs1-3 comprising amino acids 24-37, 53-59, and 92-102 of SEQ ID NO:7, respectively, and heavy chain CDRs1-3 comprising amino acids 31-35, 50-65, and 99-107 of SEQ ID NO:8, respectively.
In embodiment 21 is provided the method according to embodiment 20, wherein the presence of HT-2 toxin is determined using an immunoassay.
In embodiment 22 is provided the method according to embodiment 21, wherein the immunoassay is a non-competitive, homogenous immunoassay.
In embodiment 23 is provided the method according to embodiment 22, wherein the non-competitive homogenous immunoassay is a time resolved fluorescent resonance energy transfer (TR-FRET) assay.
In embodiment 24 is provided the method according to any one of embodiments 20 to 23, wherein said immunoassay is selected from the group consisting of a radioimmunoassay, an enzyme-linked immunosorbent assay, a lateral flow assay, a particle agglutination assay, a sandwich assay, a protein chip assay, an enzyme immunoassay, an antigen capture immunoassay, a fluorescence immunoassay, and a luminescent immunoassay.
In embodiments 25 is provided the method according to any one of embodiments 21 to 24, wherein said immunoassay is in a direct assay format.
In embodiment 26 is provided the method according to any one embodiments 20 to 25, wherein the sample is selected from the group of a food sample, feed sample, food raw material sample, feed raw material sample, environmental sample, and a sample originating from a human, or an animal, or a microbe.
In embodiment 27 is provided the method according to any one of embodiments 21 to 26, wherein the immunoassay is used in a sensor.
In embodiment 28 is provided use of the binding partner according to any one of embodiments 1 to 5 in a sensor.
In embodiment 29 is provided an antibody clone, which comprises an antibody according to embodiment 2 and deposited with VTT Culture Collection under accession number VTT E-143343.
In embodiment 30 is provided an antibody clone, which comprises an antibody according to embodiment 8 and deposited with VTT Culture Collection under accession number VTT E-143342.

### EXAMPLES

### Example 1

### Construction of the antibody gene library

Mice were immunized with HT-2 toxin (Sigma Aldrich) - Blue protein (Thermo Fisher Scientific) conjugate provided by Verifin (Finnish Institute for Verification of the Chemical Weapons Convention) in Freund's adjuvant. The mouse showing the best immune response was selected to be the source for the construction of the antibody gene library displayed on bacteriophages.

Homogenization of the mouse spleen was done using sterilized Kinematica AG Polytron PT 1200-homogenizator. The total RNA was extracted from homogenized spleen using a commercial Qiagen RNeasy® Midi/Maxi-kit according to manufacturer's instructions. In addition, on column DNase digestion was carried out using RNase-free DNase set (Qiagen).

Complementary DNA (cDNA) was synthesized from template totRNA using Phusion® RT-PCR kit (Finnzymes) with oligo-dT priming.

The mouse IgG light chain and heavy chain were cloned separately from the template cDNA. Both light and heavy variable domains were amplified by PCR using specific primers for heavy and light variable chains and constant regions. The primer sequences were as described in WO2004/046733. An antibody fragment (Fab) phage display library was constructed as described in WO2004/046733.

Electrotransformation was made with 1 µg of validated phagemid library DNA and 200µL of XL-1 blue cells (Stratagene). 3 x 1 mL of 37°C SOC-solution was added to harvest the transformed cells from the electroporation cuvettes and the transformed cells were incubated for 1 hour at 37°C with rotation. 7 mL of 37°C SB-medium with 20 µg/mL carbenicillin and 10 µg/mL tetracycline was added and the total of 10 mL cultivation was incubated for 1 h at 37°C with shaking. Carbenicillin was added to final concentration of 50 µg/mL and incubation was continued for 1 h at 37°C with shaking. 1 mL of helper phage VCS M13 (Stratagene) with a titer of 7 · 10¹¹ pfu/mL was added to the XL1-blue culture and incubated for 15-30 minutes at 37°C without shaking to allow the infection. The culture was transferred into 90 mL of SB-media including 50 mg/mL carbenicillin and 10 µg/mL of tetracycline and incubated for 2 hours at 37°C with shaking. Finally kanamycin (Sigma-Aldrich) was added to final concentration of 70 µg/mL and incubated at 34°C with shaking overnight.

The phages were isolated by centrifuging the cells at 4000 rpm for 15 minutes at 4°C followed by the addition of 25 mL of pre-cooled 20% PEG6000 - 2,5M NaCl. The phages were precipitated on ice for 30 min, centrifuged (9000 rpm) for 20 min at 4°C and the supernatant was discarded. The pellet containing the phages was resuspended in 2 mL of PBS and centrifuged in two 1 mL portions for 5 min at 14 000 rpm. The precipitation was repeated by adding 250 µL of pre-cooled 20% PEG6000 - 2,5M NaCl / 1 mL of supernatant, precipitated on ice for 30 min, centrifuged at 11 000 rpm for 10 min, the supernatant was discarded and the pellet was resuspended in 1 mL of PBS. The library was pooled after precipitation.

### Example 2

### Development of anti HT-2 toxin antibody

The magnetic beads (Invitrogen, M-270 Epoxy Dynabeads®) were coated with HT-2 alkaline phosphatase protein conjugate (provided by Verifin) according to the instructions from the manufacturer. The beads were incubated for 24 h in a rotator (Biosan Biorotator RS-multi). After the coating the beads were washed four times with PBS and resuspended in 50 µL of PBS. The amount of the bound HT-2 protein conjugate was determined by Micro-BCA protocol (Thermo Scientific, Pierce® BCA Protein assay kit). The magnetic beads were blocked with 1% milk-PBS solution (pH 7.3) and incubated for one hour in a rotator. After washing and re-suspension to PBS, the beads were ready for use in selection.

The selection of the anti-HT-2 toxin antibodies was done with a magnetic bead processor (KingFisher, ThermoFisher Scientific) utilising the following protocol: 200 µL of phage library was added to the HT-2 toxin- AP protein coated magnetic beads together with 20 µg soluble AP-protein and 100µL PBS-Tween20 (0,05%). Bead solution was incubated overnight in a rotator and then washed 5-6 times for 20 seconds with PBS-Tween20 (0.05%). Bound phages were eluted with TEA (triethylamine, Sigma-Aldrich) buffer pH 11.75 and neutralized with 6.9 µL of 1M HCl before infecting 900 µL bacterial XL-1 blue cells. Antibody displaying phages for the second round were made as previously mentioned. Totally four rounds of selection were performed with this protocol except the amount of beads was decreased by 1 µL on each round. The DNA coding the Fab-fragments from each round was cloned to pKKTac production vector (as described in WO2004/046733) and the individual clones were cultivated in 96 well plates and the Fab-fragments produced were screened for their binding properties by ELISA.

Colonies were picked and transferred to growth media (100 µL of SB including 100 µg/mL ampicillin, 10 ug/mL tetracycline and + 1% Glucose / well) from LB-amp plates using Genetix QPix robotic colony picker. The plates were incubated overnight with shaking at 37°C and 80% humidity.

200 µL of induction media (SB including 100 µg/mL ampicillin, 10 µg/mL tetracyclin, 0.1% glucose and 1mM IPTG) was dispensed on 96 well plates and 9 µL of fresh cell culture was inoculated on induction plates. The plates were incubated again overnight with shaking at 37°C and 80% humidity.

MaxiSorp-plates (NUNC) were coated with 100 ng of HT2-toxin-Blue protein conjugates in 100µL of 0.1 M Na-bicarbonate buffer (pH 9.6) to each well overnight at 4°C. The plates were blocked with Superblock Blocking Buffer (Thermo Scientific) with 0.05 % Tween 20 (Sigma Life Sciences). 100µL of culture supernatants were applied to each well and incubated for an hour. After 3 x 200 µL PBS washes 100 µL of the detection antibody (Goat anti-mouse-kappa IgG-Alkaline phosphatase, Southern Biotech) was diluted 1:2000 to 50% Super Block-solution was added to each well. Detection solution was 2 mg of PNNP in 1 mL of diethanolamine-MgCl₂-buffer and after 30 minute incubation time the A₄₀₅ was measured.

The best clones from the primary screen were subjected to competitive ELISA and the HT2-10 Fab was selected for further analysis. Competitive ELISA was performed as described previously except the 1 hour pre-incubation with free HT-2 and T-2 toxins in final concentrations of 1 µM, 100 nM, 10 nM, 1 nM and 100 pM, as well as 0 pM as a control sample) The clone HT2-10 showed similar inhibition for both HT-2 and T-2 toxins.

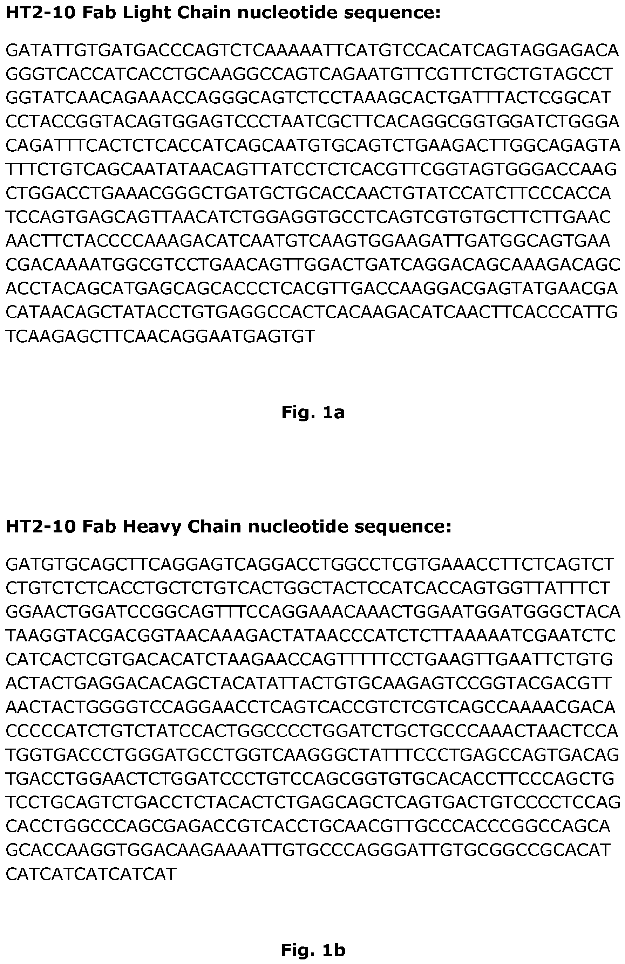

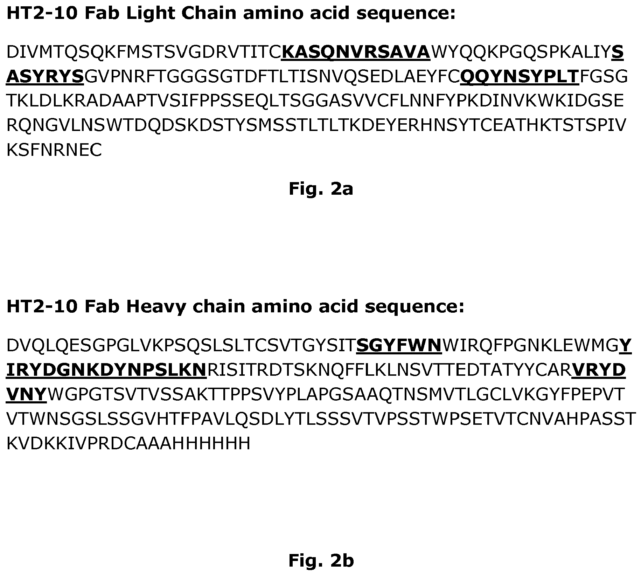

The nucleotide sequence of HT2-10 Fab light chain is set forth as SEQ ID NO:1 (Fig. 1a), and the amino acid sequence of HT2-10 Fab light chain is set forth as SEQ ID NO:3. CDRs LCDR1, LCDR2 and LCDR3 of the HT2-10 Fab light chain correspond to amino acids 24-34, 50-56, and 89-97 of SEQ ID NO:3 (Fig. 2a).

The nucleotide sequence of HT2-10 Fab heavy chain is set forth as SEQ ID NO:2 (Fig. 1b), and the amino acid sequence of HT2-10 Fab heavy chain is set forth as SEQ ID NO:4. CDRs HCDR1, HCDR2 and HCDR3 of the HT2-10 Fab heavy chain correspond to amino acids 31-36, 51-66, and 99-105 of SEQ ID NO:4 (Fig. 2b).

The amino acid sequence of HT2-10 Fab heavy chain (SEQ ID NO:4) is with 6His-Tag.

### Example 3

### Development of the anti-immune complex antibody for HT2-10 antibody and HT-2 toxin

The immune complex antibodies were selected from VTT naïve Human scFv phage display library (the library was constructed as described in WO2004/046733). The library was displayed on the surface of M13 Hyperphages (Progen).

BcMag IDA-modified magnetic beads (Bioclon Cat#FF-106 300 mg) were charged with cobalt (Co⁺²) and purified HT2-10 Fab fragments were immobilized via the six histidine tag in oriented manner onto the beads. The immobilization was carried out according to manufacturer's instructions followed by oxidation using H₂O₂. (Hale 1995). The final concentration was 3.7 mg Fab/ 30 mg magnetic beads.

The selection was done using automated magnetic bead processor (King Fisher). First the magnetic beads containing anti-HT2 toxin Fab clone 10 (HT2-10) were incubated for 60 min with 25 µM solution of free HT-2 toxin in 1 % DMSO-PBST. After incubation the beads were washed for 2 seconds and then incubated for 1 hour with the antibody library. After incubation the beads were washed 3 times with 0.05 % PBST (30 sec) and eluted for 30 min with Glycine-HCl pH 1.5. The eluted phages were neutralised with 15 µL of 1 M Tris and used for infection for the next selection round.

For the following rounds the output phages from the first round were diluted to the concentration of 2.5 x 10¹⁰ phages/400 µL PBST. The same amount of free hapten was used in each round. The multivalent display phages were used on the first three rounds while the monovalent display was utilised on the last round of selection. On the second round a soluble anti-hapten Fab was used to deplete the binders for the common constant region of the Fab-fragments. The third and fourth rounds were of selection were depleted with immobilized anti-HT2-10 clone without HT-2 toxin.

After the incubation the beads were washed three times with buffer PBST (2 min) and eluted 30 min with Glycine-HCl pH 1.5.

Phage ELISA was used to choose the best selection round having the highest relation between the wells containing the hapten containing and the blank wells. Single phage clones were tested from the best selection round and the best clones were sequenced. The best one anti-IC-HT2-10 (H5) was synthetized as a Fab fragment (DNA 2.0). The corresponding single chain antibody (scFv) was cloned to a production vector containing the alkaline phosphatase as a fusion partner.

Antibodies and the alkaline phosphatase fusion protein contain six histidine tags for purification and iimmobilization purposes. They were produced in *Escherichia coli* bacteria (anti HT2-10 in pKKTac plasmid in RV308 strain (ATCC 31608), anti-IC HT2-10 (H5) in pJExpress (DNA2.0) plasmid in RV308 strain, anti-IC HT2-10 (scFv H5) - AP fusion in pJExpress plasmid in RV308 strain All antibodies were purified by metal affinity chromatography according to the manufacturer's instructions (GE Healthcare).

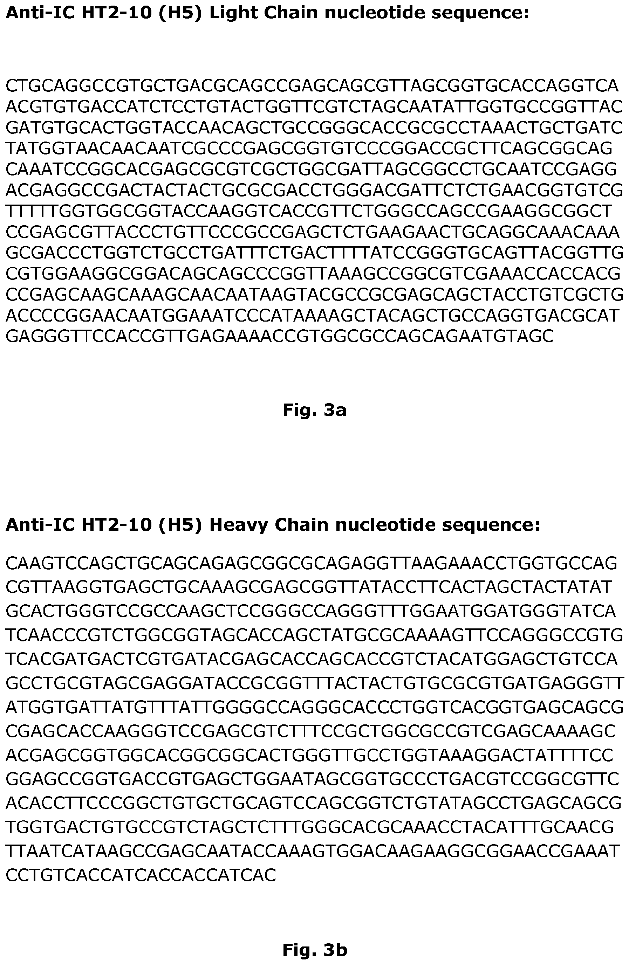

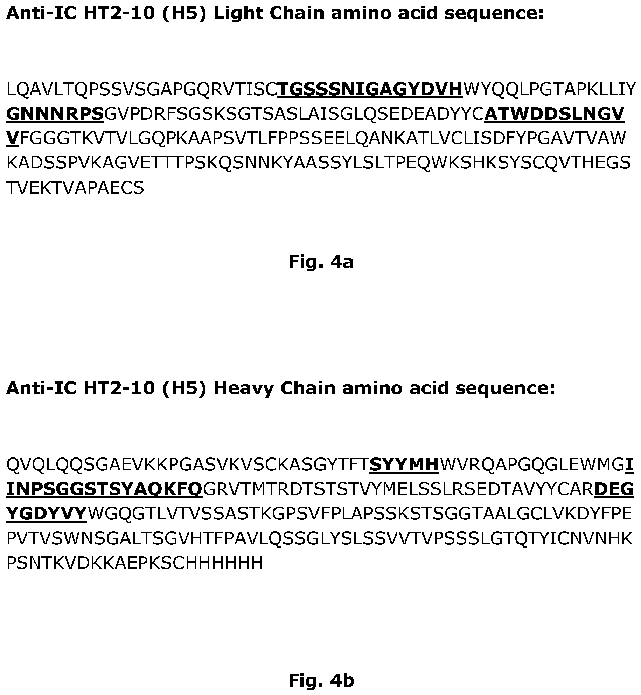

The nucleotide sequence of anti-IC-HT2-10 (H5) light chain is set forth as SEQ ID NO:5 (Fig. 3a), and the amino acid sequence of anti-IC-HT2-10 (H5) light chain is set forth as SEQ ID NO:7. CDRs CDR1, CDR2 and CDR3 of the anti-IC-HT2-10 (H5) light chain correspond to amino acids 24-37, 53-59, 92-102 of SEQ ID NO:7 (Fig. 4a).

The nucleotide sequence of anti-IC-HT2-10 (H5) heavy chain is set forth as SEQ ID NO:6 (Fig. 3b), and the amino acid sequence of anti-IC-HT2-10 (H5) heavy chain is set forth as SEQ ID NO:8. CDRs CDR1, CDR2 and CDR3 of the anti-IC-HT2-10 (H5) heavy chain correspond to amino acids 31-35, 50-65 and 99-107 of SEQ ID NO:8 (Fig. 4b).

The amino acid sequence of anti-IC-HT2-10 Fab heavy chain (SEQ ID NO:8) is with 6His-Tag.

### Example 4

### Single step immune complex assay (FRET)

The immune complex antibody pair was labelled with different fluorescent labels. The primary antibody HT2-10 was labelled with Europium chelate (Kaivogen Loisto615). 0.1 mg Europium ITC chelate was dissolved in DDIW, mixed with 0.3 mg of primary antibody HT2-10 and rotated over-night at 4°C. The buffer of the conjugation mixture was changed to 50 mM Tris pH 7.8; 0.9 % NaCl using NAP5 columns (GE Healthcare). The conjugation degree was determined using Europium standard (Perkin Elmer) according to instructions from the manufacturer (Perkin Elmer Lance® kit).

The anti-immune complex antibody H5 was labelled with Alexa Fluor 647 label using Alexa Fluor protein labelling kit according to instructions by the manufacturer (Molecular probes Inc.)

### Assay principle

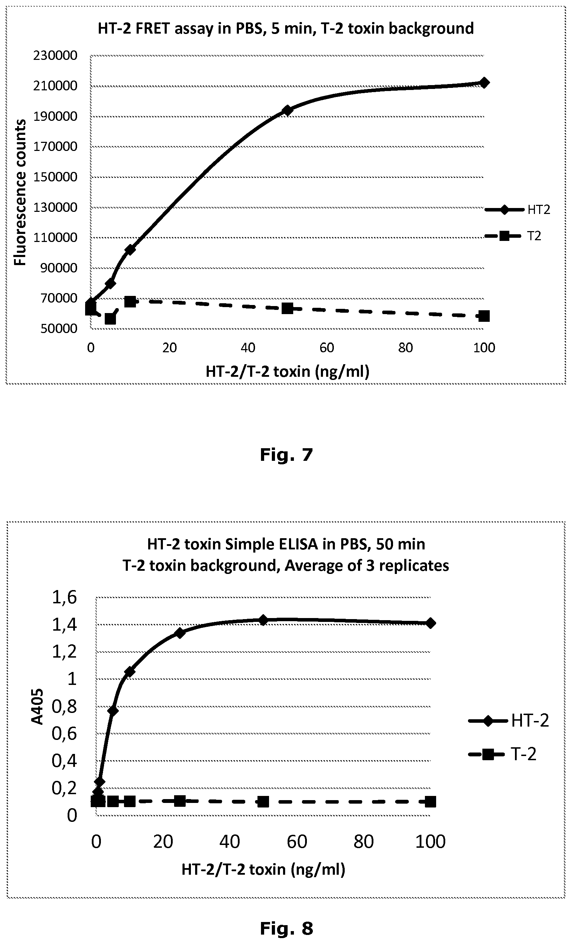

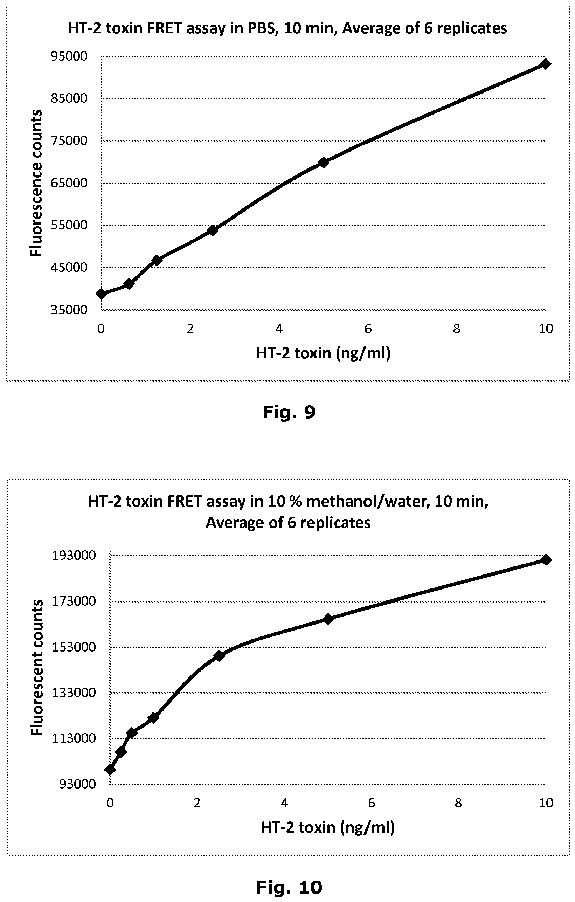

1 µg of both labelled antibodies were added to microtiter wells in 10 µL volume (diluted in PBS) and 80 µL of sample was pipetted on the reagents. The specificity was determined by spiking PBS pH 7.4 with HT-2 toxin and T-2 toxin concentrations of 0, 5, 10, 50 and 100 ng/mL (Figure 7). The linearity of the assay was determined by spiking PBS or 10% methanol/water with 0; 0.25; 0.5; 1.0; 2.5; 5.0 and 10 ng/mL concentrations of HT-2 toxin (Figures 9 and 10 for PBS and 10% methanol/water, respectively). After adding the sample the plate was shaken shortly and measured using Victor V fluorometer (Perkin Elmer Wallac) after 5 mins (excitation at 340 nm and detection at 665 nm).

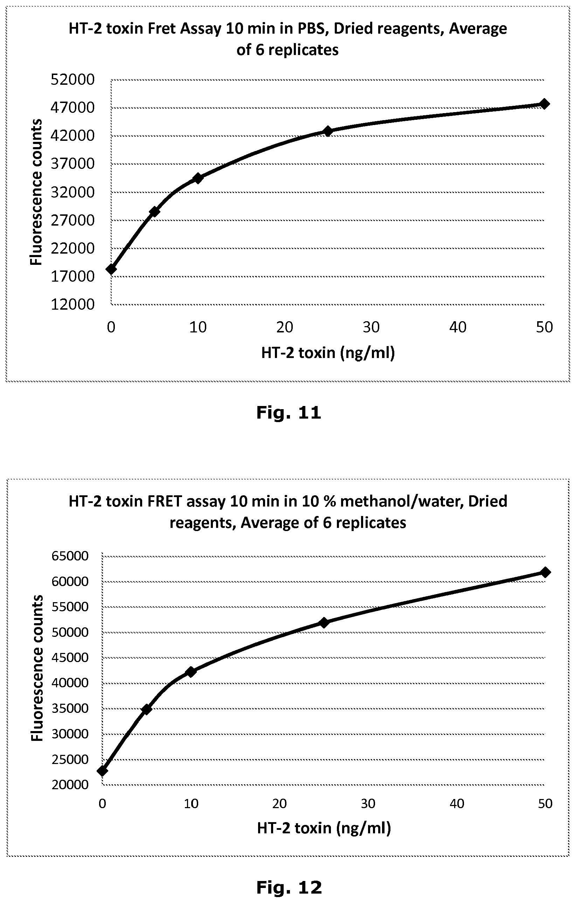

The labelled antibodies can also be dried on the bottom of the assay plate. In this fastest and simplest format the user only adds the sample and measures the plate after few minutes (Figure 11 and 12).

### Example 5

### Simple ELISA assay (AP)

The anti- HT2/T2 Fab(10) was biotinylated by adding 3 x molar excess of Sulfo-NHS-SS-Biotin in PBS and incubated for 30 min with rotation according to the manufacturer's instructions (Thermo Scientific Pierce). The biotinylated anti- HT2/T2 Fab(10) was purified by Econopac (Bio-Rad). The protein concentrations were measured from the elution fractions by Nanodrop 1000 (Thermo Scientific) and the fractions having more than 0.3 mg/mL protein were pooled. The success of the biotinylation was checked with immobilizing 500 ng/well of the biotinylated primary antibody to the streptavidin plate (Kaivogen) and adding HT-2 toxin AP conjugate 500 ng/well. As a control, non-biotinylated anti-HT2/T2 Fab(10) was used. After washing three times with PBS the AP substrate 2 mg/mL PNPP (Sigma) in Diethanolamine-MgCl₂ buffer (Reagena) was added and the amount of bound HT2-AP was detected by measuring the A405.

The pJExpress401 vector with alkaline phosphatase fusion protein was ordered from DNA2.0.

The anti-immune complex clone anti-IC-HT2-10(H5) was cloned into the AP-vector by digesting the whole single chain construct from phagemid vector (NcoI/NotI) and ligating it into 2 x NcoI/NotI digested pJExpress401 AP vector.

The scFv-AP fusion was produced in bacterial cells in small scale bottle cultivation and was purified by metal affinity chromatography according to the manufacturer's instructions (GE Healthcare)

### The assay principle:

The biotinylated primary antibody HT2-10 is immobilized on the surface of the streptavidin coated 96-well microtiter plates (Kaivogen Kaisa96) for 30 mins. 200µL of 0.2 µg/mL free biotin was used as a blocking solution. The 50 µL sample including the analyte HT-2 toxin is mixed with 50 µL secondary antibody H5 and pipetted on the well at the same time and incubated for 1 h in RT in mild shaking. After three washes with 200µL PBS-buffer the substrate for AP is added (2 mg/mL of PNPP (Sigma) in Ethanolamine-MgCl₂ (Reagena)) and the absorbance A405 is measured.

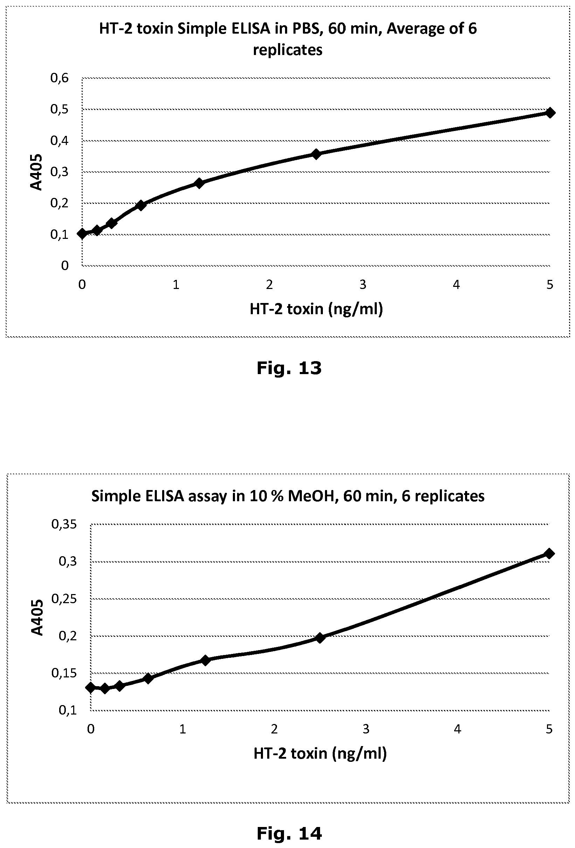

The AP assay is specific to HT-2 toxin without detectable cross-reactivity to T-2 toxin. (Figure 13). The simple Elisa assay using scFv-AP fusion can be applied also to samples containing 10% methanol (Figure 14).

### Example 6

### Lateral flow assay for HT-2 toxin

The anti- HT2-10 Fab was coupled to 40 nm gold nanoparticles by adding 2.5 µg/mL of Fab to colloidal gold solution (BBI solutions) with 3 % of BSA (Sigma). After 20 minutes of incubation at room temperature in rotation the unbound antibody was separated from the conjugated Fab fragments by centrifugation for 15 min at 8000 g and by washing the conjugate once with sterile distilled water (MilliQ). The gold nanoparticles containing anti-HT2-10 Fab were resuspended in 1 % BSA-water after another centrifugation for 15 min at 8000 g.

For the test line, the anti-IC HT2-10 Fab (H5) (1 mg/mL) and for the control line, Goat anti-mouse IgG (Fab specific) antibody (Sigma) (1 mg/mL) were sprayed on a preblocked nitrocellulose membrane (MDI Membrane Technologies) (300 mm x 20 mm) using ZX1000 and Air Jet Quanti instruments (BioDot).

The sprayed membranes were dried over-night at 24 °C and assembled onto a backing card with a sample wick (300 mm x 27 mm, Whatman CF5) overlapping 1 mm. The 4.5 mm test strips were cut using a CM4000 guillotine cutter (BioDot).

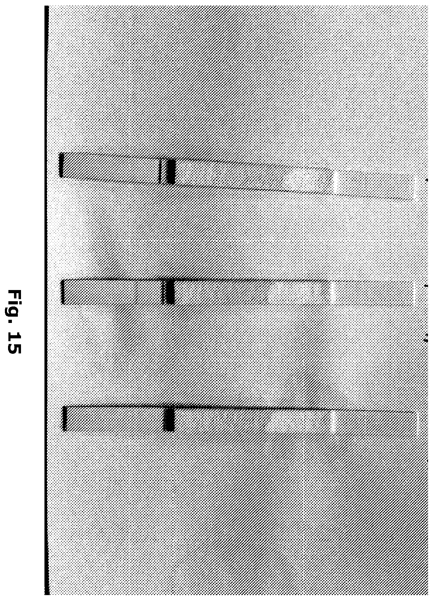

The primary antibody anti-HT2-10 Fab labelled with colloidal gold was mixed in a non-treated microtiter well with the running buffer and the sample. The LFA strip was introduced to a microtiter well and the result was read after 10 minutes. The red colour in the test and control lines indicates a positive sample. The red control line only indicates a negative sample and that the test strip is valid. These results show that LFA is specific to HT-2 toxin and doesn't cross-react with T-2 toxin (Figure 15.)

### References

Dzantiev, B.B., Byzova, N.A., Urusov, A.E. and Zherdev, A.V. (2014) Trends in Analytical Chemistry 55: 81-93.

Gonzales-Techera, A., Vanrell, L., Hammock, B.D. and Gonzales-Sapienza, G. (2007) Anal. Chem 79:7799-7806.

Hale J. E (1995) Analytical biochemistry 231: 46-49.

Hara et al. (2013) Analytica Chimica Acta 793:107-113.

Hemmilä I. et al., (1988), Clin. Chem. 34:2320-2322.

Kabat et al., (1991) Sequences of Proteins of Immunological Interest, 5th Ed. Public Health Service, National Institutes of Health, Bethesda, Md.

Li, T., Jeon, K-S., Suh, Y.D. and Kim, M.-G. (2011) Chem. Commun. 47:9098-9100.

Li, P., Zhang, Z., Hu, X. and Zhang Q. (2013) Mass Spectrometry Reviews 32: 420-452.

Li, T., Byun, J-Y., Kim, B.B., Shin, Y.-B. and Kim, M.-G. (2013) Biosensors and Bioelectronics 42: 403-408.

Ueda et al. (1996) Vanrell, L., Gonzales-Techera, A., Hammock, B.D. and Gonzalez-Sapienza, G. (2013) Analytical Chemistry 85: 1177-1182.

Commission recommendation of 27 March 2013 on the presence ofTt-2 and HT-2 toxin in cereals and cereal products (2013/165/EU).

## Claims

1. A binding partner recognizing an immune complex between HT-2 toxin and anti-HT-2 toxin antibody and comprising light chain CDRs1-3 comprising amino acids 24-37, 53-59, and 92-102 of SEQ ID NO:7, respectively, and heavy chain CDRs1-3 comprising amino acids 31-35, 50-65, and 99-107 of SEQ ID NO:8, respectively.

2. The binding partner according to claim 1, wherein the binding partner is an antibody or an antibody fragment.

3. The binding partner according to claim 1 or 2, wherein the binding partner forms an antibody fragment Fab, scFv, or a recombinant monoclonal antibody.

4. The binding partner according to any one of claims 1 or 3, wherein the binding partner comprises amino acid sequences of SEQ ID NO:7 and SEQ ID NO:8.

5. A test-kit comprising the binding partner according to any one of claims 1 to 4.

6. The test-kit according to claim 5, further comprising another binding partner comprising light chain CDRs1-3 comprising amino acids 24-34, 50-56, and 89-97 of SEQ ID NO:3, respectively, and heavy chain CDRs1-3 comprising amino acids 31-36, 51-66, and 99-105 of SEQ ID NO:4, respectively.

7. The test kit according to claim 6, wherein said another binding partner is an antibody or an antibody fragment.

8. The test kit according to claim 6 to 7, wherein said another binding partner forms an antibody fragment Fab, scFv, or a recombinant monoclonal antibody.

9. The test-kit according to any one of claims 6 to 8, wherein said another binding partner specifically recognizes HT-2 toxin, or both HT-2 toxin and T-2 toxin.

10. The test kit according to any one of claims 6 to 9, comprising reagents for a non-competitive, homogenous immunoassay.

11. The test kit according to any one of claims 6 to 10, comprising reagents for time resolved fluorescent resonance energy transfer (TR-FRET) assay.

12. A polynucleotide encoding the binding partner according to any one of claims 1 to 4.

13. An isolated host cell that has been transformed with a polynucleotide encoding the binding partner according to any one of claims 1 to 4 and being capable of expressing the binding partner according to any one of claims 1 to 4.

14. Use of the binding partner according to any one of claims 1 to 4 for detecting an analyte in a food or animal feed sample.

15. Use of the binding partner according to any one of claims 1 to 4 for detecting an analyte in a sample taken from a human or an animal exposed to a HT-2 toxin.

16. Use of the binding partner according to any one of claims 1 to 4 for detecting an analyte in an environmental sample.

17. A method for detecting HT-2 toxin in a sample, comprising
- reacting the sample with a reagent pair comprising a first binding partner that binds to a HT-2 toxin or T-2 toxin in the sample to form a complex, and a second binding partner that specifically binds to said complex; and
- determining the binding of the second binding partner to the complex, thereby indicating presence of the HT-2 toxin in the sample,
wherein said first binding partner comprises light chain CDRs1-3 comprising amino acids 24-34, 50-56, and 89-97 of SEQ ID NO:3, respectively, and heavy chain CDRs1-3 comprising amino acids 31-36, 51-66, and 99-105 of SEQ ID NO:4, respectively, and
said second binding partner comprises light chain CDRs1-3 comprising amino acids 24-37, 53-59, and 92-102 of SEQ ID NO:7, respectively, and heavy chain CDRs1-3 comprising amino acids 31-35, 50-65, and 99-107 of SEQ ID NO:8, respectively.

18. The method according to claim 17, wherein the presence of HT-2 toxin is determined using an immunoassay.

19. The method according to claim 18, wherein the immunoassay is a non-competitive, homogenous immunoassay.

20. The method according to claim 19, wherein the non-competitive homogenous immunoassay is a time resolved fluorescent resonance energy transfer (TR-FRET) assay.

21. The method according to any one of claims 17 to 20, wherein said immunoassay is selected from the group consisting of a radioimmunoassay, an enzyme-linked immunosorbent assay, a lateral flow assay, a particle agglutination assay, a sandwich assay, a protein chip assay, an enzyme immunoassay, an antigen capture immunoassay, a fluorescence immunoassay, and a luminescence immunoassay.

22. The method according to any one of claims 18 to 21, wherein said immunoassay is in a direct assay format.

23. The method according to any one of claims 19 to 22, wherein the sample is selected from the group of a food sample, feed sample, food raw material sample, feed raw material sample, environmental sample, and a sample originating from a human, or an animal, or a microbe.

24. The method according to any one of claims 20 to 23, wherein the immunoassay is used in a sensor.

25. Use of the binding partner according to any one of claims 1 to 4 in a sensor.

26. An antibody according to claim 2 produced in *Escherichia coli* bacteria clone deposited with VTT Culture Collection under accession number VTT E-143343.

27. The test kit according to claim 7, wherein said antibody is produced in *Escherichia coli* bacteria clone deposited with VTT Culture Collection under accession number VTT E-143342.

## Patentansprüche

1. Bindungspartner, der einen Immunkomplex zwischen HT-2-Toxin und Anti-HT-2-Toxin-Antikörpern erkennt und leichtkettige CDRs1-3, die Aminosäuren 24-37, 53-59 beziehungsweise 92-102 der SEQ ID NO:7 aufweisen, und schwerkettige CDRs1-3, die Aminosäuren 31-35, 50-65 beziehungsweise 99-107 der SEQ ID NO:8 aufweisen, umfasst.

2. Bindungspartner nach Anspruch 1, wobei der Bindungspartner ein Antikörper oder ein Antikörperfragment ist.

3. Bindungspartner nach Anspruch 1 oder 2, wobei der Bindungspartner ein Antikörperfragment Fab, scFv, oder einen rekombinanten monoklonalen Antikörper bildet.

4. Bindungspartner nach einem der Ansprüche 1 oder 3, wobei der Bindungspartner Aminosäuresequenzen der SEQ ID NO:7 und SEQ ID NO:8 umfasst.

5. Testkit, das den Bindungspartner nach einem der Ansprüche 1 bis 4 umfasst.

6. Testkit nach Anspruch 5, ferner umfassend: einen anderen Bindungspartner, der leichtkettige CDRs1-3, die Aminosäuren 24-34, 50-56 beziehungsweise 89-97 der SEQ ID NO:3 aufweisen, und schwerkettige CDRs1-3, die Aminosäuren 31-36, 51-66 beziehungsweise 99-105 der SEQ ID NO:4 aufweisen, umfasst.

7. Testkit nach Anspruch 6, wobei der andere Bindungspartner ein Antikörper oder ein Antikörperfragment ist.

8. Testkit nach Anspruch 6 bis 7, wobei der andere Bindungspartner ein Antikörperfragment Fab, scFv, oder einen rekombinanten monoklonalen Antikörper bildet.

9. Testkit nach einem der Ansprüche 6 bis 8, wobei der andere Bindungspartner speziell HT-2-Toxin oder sowohl HT-2-Toxin als auch T-2-Toxin erkennt.

10. Testkit nach einem der Ansprüche 6 bis 9, das Reagenzien für einen nichtkompetitiven, homogenen Immunassay umfasst.

11. Testkit nach einem der Ansprüche 6 bis 10, das Reagenzien für einen zeitauflösenden Fluoreszenz-Resonanz-Energietransfer-Assay (TR-FRET) umfasst.

12. Polynukleotid, das den Bindungspartner nach einem der Ansprüche 1 bis 4 kodiert.

13. Isolierte Wirtszelle nach deren Transformation mit einem Polynukleotid, das den Bindungspartner nach einem der Ansprüche 1 bis 4 kodiert, und mit Fähigkeit zur Expression des Bindungspartners nach einem der Ansprüche 1 bis 4.

14. Verwendung des Bindungspartners nach einem der Ansprüche 1 bis 4 zum Bestimmen eines Analyts in einer Nahrungsmittel- oder Tierfuttermittelprobe.

15. Verwendung des Bindungspartners nach einem der Ansprüche 1 bis 4 zum Bestimmen eines Analyts in einer Probe, die einem Menschen oder einem Tier entnommen wird, der bzw. das einem HT-2-Toxin ausgesetzt war.

16. Verwendung des Bindungspartners nach einem der Ansprüche 1 bis 4 zum Bestimmen eines Analyts in einer Umweltprobe.

17. Verfahren zum Erfassen von HT-2-Toxin in einer Probe, umfassend:
- Reaktion der Probe mit einem Reagenspaar, das einen ersten Bindungspartner, der sich an ein HT-2-Toxin oder T-2-Toxin in der Probe bindet und einen Komplex ausbildet, und einen zweiten Bindungspartner, der sich speziell an den Komplex bindet, umfasst; und
- Bestimmen der Bindung des zweiten Bindungspartners am Komplex, dadurch Nachweisen des Vorliegens von HT-2-Toxin in der Probe,
wobei der erste Bindungspartner leichtkettige CDRs1-3, die Aminosäuren 24-34, 50-56 beziehungsweise 89-97 der SEQ ID NO:3 aufweisen, und schwerkettige CDRs1-3, die Aminosäuren 31-36, 51-66 beziehungsweise 99-105 der SEQ ID NO:4 aufweisen, umfasst, und
der zweite Bindungspartner leichtkettige CDRs1-3, die Aminosäuren 24-37, 53-59 beziehungsweise 92-102 der SEQ ID NO:7 aufweisen, und schwerkettige CDRs1-3, die Aminosäuren 31-35, 50-65 beziehungsweise 99-107 der SEQ ID NO:8 aufweisen, umfasst.

18. Verfahren nach Anspruch 17, wobei das Vorliegen von HT-2-Toxin mit Hilfe eines Immunassays bestimmt wird.

19. Verfahren nach Anspruch 18, wobei der Immunassay ein nicht-kompetitiver, homogener Immunassay ist.

20. Verfahren nach Anspruch 19, wobei der nicht-kompetitive homogene Immunassay ein zeitauflösender Fluoreszenz-Resonanz-Energietransfer-Assay (TR-FRET) ist.

21. Verfahren nach einem der Ansprüche 17 bis 20, wobei der Immunassay gewählt ist aus der Gruppe bestehend aus Radioimmunassay, enzymgebundener Immunosorbent-Assay, Lateral-Flow-Assay, Partikelagglutinations-Assay, Sandwich-Assay, Protein-Chip-Assay, Enzymimmunassay, Antigenabfang-Immunassay, Fluoreszenz-Immunassay und Lumineszenz-Immunassay.

22. Verfahren nach einem der Ansprüche 18 bis 21, wobei der Immunassay das Format eines direkten Assays aufweist.

23. Verfahren nach einem der Ansprüche 19 bis 22, wobei die Probe gewählt ist aus der Gruppe von Lebensmittelprobe, Futtermittelprobe, Lebensmittelrohmaterialprobe, Futtermittelrohmaterialprobe, Umweltprobe und Probe mit Herkunft von einem Menschen, einem Tier oder einer Mikrobe.

24. Verfahren nach einem der Ansprüche 20 bis 23, wobei der Immunassay in einem Sensor verwendet wird.

25. Verwendung des Bindungspartners nach einem der Ansprüche 1 bis 4 in einem Sensor.

26. Antikörper nach Anspruch 2, der im Bakterienclon *Escherichia coli* erzeugt wird, welcher in der VTT Culture Collection unter der Accession-Nummer VTT E-143343 abgelegt ist.

27. Testkit nach Anspruch 7, wobei der Antikörper im Bakterienclon *Escherichia coli* erzeugt wird, der in der VTT Culture Collection unter der Accession-Nummer VTT E-143342 abgelegt ist.

## Revendications

1. Partenaire de liaison reconnaissant un complexe immun entre la toxine HT-2 et les anticorps antitoxine HT-2 et comprenant des CDRs1-3 de chaîne légère comprenant des acides aminés 24-37, 53-59 et 92-102 de SEQ ID No:7, respectivement, et des CDRs1-3 de chaîne lourde comprenant des acides aminés 31-35, 50-65 et 99-107 de SEQ ID NO:8, respectivement.

2. Partenaire de liaison selon la revendication 1, dans lequel le partenaire de liaison est un anticorps ou un fragment d'anticorps.

3. Partenaire de liaison selon la revendication 1 ou 2, dans lequel le partenaire de liaison forme un fragment d'anticorps Fab, scFv, ou un anticorps monoclonal recombinant.

4. Partenaire de liaison selon l'une des revendications 1 ou 3, dans lequel le partenaire de liaison comprend des séquences d'acide aminé de SEQ ID NO:7 et de SEQ ID NO:8.

5. Kit de test comprenant le partenaire de liaison selon l'une des revendications 1 à 4.

6. Kit de test selon la revendication 5 comprenant également un autre partenaire de liaison comprenant des CDRs1-3 de chaîne légère comprenant des acides aminés 24-34, 50-56 et 89-97 de SEQ ID No:3, respectivement, et des CDRs1-3 de chaîne lourde comprenant des acides aminés 31-36, 51-66 et 99-105 de SEQ ID NO:4, respectivement.

7. Kit de test selon la revendication 6 dans lequel ledit autre partenaire de liaison est un anticorps ou un fragment d'anticorps.

8. Kit de test selon la revendication 6 ou 7, dans lequel ledit autre partenaire de liaison forme un fragment d'anticorps Fab, scFv, ou un anticorps monoclonal recombinant.

9. Kit de test selon l'une des revendications 6 à 8, dans lequel ledit autre partenaire de liaison reconnaît spécifiquement la toxine HT-2 ou tant la toxine HT-2 que la toxine T-2.

10. Kit de test selon l'une des revendications 6 à 9 comprenant des réactifs pour un immunoessai homogène, non compétitif.

11. Kit de test selon l'une des revendications 6 à 10 comprenant des réactifs pour un essai de transfert d'énergie de fluorescence par résonance en temps résolu (TR-FRET).

12. Polynucléotide encodant le partenaire de liaison selon l'une des revendications 1 à 4.

13. Cellule hôte isolée ayant été transformée avec un polynucléotide encodant le partenaire de liaison selon l'une des revendications 1 à 4 et étant capable d'exprimer le partenaire de liaison selon l'une des revendications 1 à 4.

14. Utilisation du partenaire de liaison selon l'une des revendications 1 à 4 pour détecter un analyte dans un échantillon d'alimentation humaine ou animale.

15. Utilisation du partenaire de liaison selon l'une des revendications 1 à 4 pour détecter un analyte dans un échantillon prélevé sur un humain ou un animal exposé à une toxine HT-2.

16. Utilisation du partenaire de liaison selon l'une des revendications 1 à 4 pour détecter un analyte dans un échantillon environnemental.

17. Procédé de détection de toxine HT-2 dans un échantillon, comprenant :
- réaction de l'échantillon avec une paire de réactifs comprenant un premier partenaire de liaison qui se lie à une toxine HT-2 ou une toxine T-2 dans l'échantillon pour former un complexe, et un deuxième partenaire de liaison qui se lie spécifiquement audit complexe; et
- détermination de la liaison au complexe du deuxième partenaire de liaison et par-là indication de la présence de la toxine HT-2 dans l'échantillon,
ledit premier partenaire de liaison comprenant des CDRs1-3 de chaîne légère comprenant des acides aminés 24-34, 50-56 et 89-97 de SEQ ID No:3, respectivement, et des CDRs1-3 de chaîne lourde comprenant des acides aminés 31-36, 51-66 et 99-105 de SEQ ID NO:4, respectivement, et
ledit deuxième partenaire de liaison comprenant des CDRs1-3 de chaîne légère comprenant des acides aminés 24-37, 53-59 et 92-102 de SEQ ID No:7, respectivement, et des CDRs1-3 de chaîne lourde comprenant des acides aminés 31-35, 50-65 et 99-107 de SEQ ID NO:8, respectivement.

18. Procédé selon la revendication 17, dans lequel la présence de la toxine HT-2 est déterminée par un immunoessai.

19. Procédé selon la revendication 18, dans lequel l'immunoessai est un immunoessai homogène, non compétitif.

20. Procédé selon la revendication 19, dans lequel l'immunoessai homogène non compétitif est un essai de transfert d'énergie de fluorescence par résonance en temps résolu (TR-FRET).

21. Procédé selon l'une des revendications 17 à 20, dans lequel l'immunoessai est choisi dans le groupe constitué par le radioimmunoessai, le dosage immunoenzymatique, l'essai de flux latéral, l'immunodosage par agglutination de particules, l'essai de type sandwich, l'essai de type puce à protéines, l'immunoessai enzymatique, l'immunodosage de capture d'antigène, l'immunoessai par fluorescence et l'immunoessai par luminescence.

22. Procédé selon l'une des revendications 18 à 21, dans lequel l'immunoessai est dans un format d'essai direct.

23. Procédé selon l'une des revendications 19 à 22, dans lequel l'échantillon est choisi dans le groupe constitué par un échantillon d'alimentation humaine, un échantillon d'alimentation animale, un échantillon de matière première d'alimentation humaine, un échantillon de matière première d'alimentation animale, un échantillon environnemental et un échantillon originaire d'un humain ou d'un animal ou d'un microbe.

24. Procédé selon l'une des revendications 20 à 23, dans lequel l'immunoessai est utilisé dans un capteur.

25. Utilisation du partenaire de liaison selon l'une des revendications 1 à 4 dans un capteur.

26. Anticorps selon la revendication 2 produit dans le clone bactérien *Escherichia coli* déposé chez la VTT Culture Collection sous le numéro d'entrée VTT E-143343.

27. Kit de test selon la revendication 7 dans lequel l'anticorps est produit dans le clone bactérien *Escherichia coli* déposé chez la VTT Culture Collection sous le numéro d'entrée VTT E-143342.


### SEQUENCE LISTING

<110> VTT Technical Research Centre
<120> Immunoassay
<130> B4092PFI
<160> 10
<170> PatentIn version 3.5
<210> 1
   <211> 642
   <212> DNA
   <213> Mus musculus
<400> 1
   gatattgtga tgacccagtc tcaaaaattc atgtccacat cagtaggaga cagggtcacc      60
   atcacctgca aggccagtca gaatgttcgt tctgctgtag cctggtatca acagaaacca     120
   gggcagtctc ctaaagcact gatttactcg gcatcctacc ggtacagtgg agtccctaat     180
   cgcttcacag gcggtggatc tgggacagat ttcactctca ccatcagcaa tgtgcagtct     240
   gaagacttgg cagagtattt ctgtcagcaa tataacagtt atcctctcac gttcggtagt     300
   gggaccaagc tggacctgaa acgggctgat gctgcaccaa ctgtatccat cttcccacca     360
   tccagtgagc agttaacatc tggaggtgcc tcagtcgtgt gcttcttgaa caacttctac     420
   cccaaagaca tcaatgtcaa gtggaagatt gatggcagtg aacgacaaaa tggcgtcctg     480
   aacagttgga ctgatcagga cagcaaagac agcacctaca gcatgagcag caccctcacg     540
   ttgaccaagg acgagtatga acgacataac agctatacct gtgaggccac tcacaagaca     600
   tcaacttcac ccattgtcaa gagcttcaac aggaatgagt gt                        642
<210> 2
   <211> 681
   <212> DNA
   <213> Mus musculus
<400> 2
   gatgtgcagc ttcaggagtc aggacctggc ctcgtgaaac cttctcagtc tctgtctctc    60
   acctgctctg tcactggcta ctccatcacc agtggttatt tctggaactg gatccggcag   120
   tttccaggaa acaaactgga atggatgggc tacataaggt acgacggtaa caaagactat   180
   aacccatctc ttaaaaatcg aatctccatc actcgtgaca catctaagaa ccagtttttc   240
   ctgaagttga attctgtgac tactgaggac acagctacat attactgtgc aagagtccgg   300
   tacgacgtta actactgggg tccaggaacc tcagtcaccg tctcgtcagc caaaacgaca   360
   cccccatctg tctatccact ggcccctgga tctgctgccc aaactaactc catggtgacc   420
   ctgggatgcc tggtcaaggg ctatttccct gagccagtga cagtgacctg gaactctgga   480
   tccctgtcca gcggtgtgca caccttccca gctgtcctgc agtctgacct ctacactctg   540
   agcagctcag tgactgtccc ctccagcacc tggcccagcg agaccgtcac ctgcaacgtt   600
   gcccacccgg ccagcagcac caaggtggac aagaaaattg tgcccaggga ttgtgcggcc   660
   gcacatcatc atcatcatca t                                              681
<210> 3
   <211> 214
   <212> PRT
   <213> Mus musculus
<400> 3
   Asp Ile Val Met Thr Gln Ser Gln Lys Phe Met Ser Thr Ser Val Gly
   1               5                   10                  15
   Asp Arg Val Thr Ile Thr Cys Lys Ala Ser Gln Asn Val Arg Ser Ala
               20                  25                  30
   Val Ala Trp Tyr Gln Gln Lys Pro Gly Gln Ser Pro Lys Ala Leu Ile
           35                  40                  45
   Tyr Ser Ala Ser Tyr Arg Tyr Ser Gly Val Pro Asn Arg Phe Thr Gly
       50                  55                  60
   Gly Gly Ser Gly Thr Asp Phe Thr Leu Thr Ile Ser Asn Val Gln Ser
   65                  70                  75                  80
   Glu Asp Leu Ala Glu Tyr Phe Cys Gln Gln Tyr Asn Ser Tyr Pro Leu
                   85                  90                  95
   Thr Phe Gly Ser Gly Thr Lys Leu Asp Leu Lys Arg Ala Asp Ala Ala
               100                 105                 110
   Pro Thr Val Ser Ile Phe Pro Pro Ser Ser Glu Gln Leu Thr Ser Gly
           115                 120                 125
   Gly Ala Ser Val Val Cys Phe Leu Asn Asn Phe Tyr Pro Lys Asp Ile
       130                 135                 140
   Asn Val Lys Trp Lys Ile Asp Gly Ser Glu Arg Gln Asn Gly Val Leu
   145                 150                 155                 160
   Asn Ser Trp Thr Asp Gln Asp Ser Lys Asp Ser Thr Tyr Ser Met Ser
                   165                 170                 175
   Ser Thr Leu Thr Leu Thr Lys Asp Glu Tyr Glu Arg His Asn Ser Tyr
               180                 185                 190
   Thr Cys Glu Ala Thr His Lys Thr Ser Thr Ser Pro Ile Val Lys Ser
           195                 200                 205
   Phe Asn Arg Asn Glu Cys
       210
<210> 4
   <211> 227
   <212> PRT
   <213> Mus musculus
<400> 4
   Asp Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Gly
               20                  25                  30
   Tyr Phe Trp Asn Trp Ile Arg Gln Phe Pro Gly Asn Lys Leu Glu Trp
           35                  40                  45
   Met Gly Tyr Ile Arg Tyr Asp Gly Asn Lys Asp Tyr Asn Pro Ser Leu
       50                  55                  60
   Lys Asn Arg Ile Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Arg Val Arg Tyr Asp Val Asn Tyr Trp Gly Pro Gly Thr Ser Val
               100                 105                 110
   Thr Val Ser Ser Ala Lys Thr Thr Pro Pro Ser Val Tyr Pro Leu Ala
           115                 120                 125
   Pro Gly Ser Ala Ala Gln Thr Asn Ser Met Val Thr Leu Gly Cys Leu
       130                 135                 140
   Val Lys Gly Tyr Phe Pro Glu Pro Val Thr Val Thr Trp Asn Ser Gly
   145                 150                 155                 160
   Ser Leu Ser Ser Gly Val His Thr Phe Pro Ala Val Leu Gln Ser Asp
                   165                 170                 175
   Leu Tyr Thr Leu Ser Ser Ser Val Thr Val Pro Ser Ser Thr Trp Pro
               180                 185                 190
   Ser Glu Thr Val Thr Cys Asn Val Ala His Pro Ala Ser Ser Thr Lys
           195                 200                 205
   Val Asp Lys Lys Ile Val Pro Arg Asp Cys Ala Ala Ala His His His
       210                 215                 220
   His His His
   225
<210> 5
   <211> 654
   <212> DNA
   <213> Mus musculus
<400> 5
   ctgcaggccg tgctgacgca gccgagcagc gttagcggtg caccaggtca acgtgtgacc      60
   atctcctgta ctggttcgtc tagcaatatt ggtgccggtt acgatgtgca ctggtaccaa     120
   cagctgccgg gcaccgcgcc taaactgctg atctatggta acaacaatcg cccgagcggt     180
   gtcccggacc gcttcagcgg cagcaaatcc ggcacgagcg cgtcgctggc gattagcggc     240
   ctgcaatccg aggacgaggc cgactactac tgcgcgacct gggacgattc tctgaacggt     300
   gtcgtttttg gtggcggtac caaggtcacc gttctgggcc agccgaaggc ggctccgagc     360
   gttaccctgt tcccgccgag ctctgaagaa ctgcaggcaa acaaagcgac cctggtctgc     420
   ctgatttctg acttttatcc gggtgcagtt acggttgcgt ggaaggcgga cagcagcccg     480
   gttaaagccg gcgtcgaaac caccacgccg agcaagcaaa gcaacaataa gtacgccgcg     540
   agcagctacc tgtcgctgac cccggaacaa tggaaatccc ataaaagcta cagctgccag     600
   gtgacgcatg agggttccac cgttgagaaa accgtggcgc cagcagaatg tagc           654
<210> 6
   <211> 681
   <212> DNA
   <213> Mus musculus
<400> 6
   caagtccagc tgcagcagag cggcgcagag gttaagaaac ctggtgccag cgttaaggtg      60
   agctgcaaag cgagcggtta taccttcact agctactata tgcactgggt ccgccaagct     120
   ccgggccagg gtttggaatg gatgggtatc atcaacccgt ctggcggtag caccagctat     180
   gcgcaaaagt tccagggccg tgtcacgatg actcgtgata cgagcaccag caccgtctac     240
   atggagctgt ccagcctgcg tagcgaggat accgcggttt actactgtgc gcgtgatgag     300
   ggttatggtg attatgttta ttggggccag ggcaccctgg tcacggtgag cagcgcgagc     360
   accaagggtc cgagcgtctt tccgctggcg ccgtcgagca aaagcacgag cggtggcacg     420
   gcggcactgg gttgcctggt aaaggactat tttccggagc cggtgaccgt gagctggaat     480
   agcggtgccc tgacgtccgg cgttcacacc ttcccggctg tgctgcagtc cagcggtctg     540
   tatagcctga gcagcgtggt gactgtgccg tctagctctt tgggcacgca aacctacatt     600
   tgcaacgtta atcataagcc gagcaatacc aaagtggaca agaaggcgga accgaaatcc     660
   tgtcaccatc accaccatca c                                               681
<210> 7
   <211> 218
   <212> PRT
   <213> Homo sapiens
<400> 7
   Leu Gln Ala Val Leu Thr Gln Pro Ser Ser Val Ser Gly Ala Pro Gly
   1               5                   10                  15
   Gln Arg Val Thr Ile Ser Cys Thr Gly Ser Ser Ser Asn Ile Gly Ala
               20                  25                  30
   Gly Tyr Asp Val His Trp Tyr Gln Gln Leu Pro Gly Thr Ala Pro Lys
           35                  40                  45
   Leu Leu Ile Tyr Gly Asn Asn Asn Arg Pro Ser Gly Val Pro Asp Arg
       50                  55                  60
   Phe Ser Gly Ser Lys Ser Gly Thr Ser Ala Ser Leu Ala Ile Ser Gly
   65                  70                  75                  80
   Leu Gln Ser Glu Asp Glu Ala Asp Tyr Tyr Cys Ala Thr Trp Asp Asp
                   85                  90                  95
   Ser Leu Asn Gly Val Val Phe Gly Gly Gly Thr Lys Val Thr Val Leu
               100                 105                 110
   Gly Gln Pro Lys Ala Ala Pro Ser Val Thr Leu Phe Pro Pro Ser Ser
           115                 120                 125
   Glu Glu Leu Gln Ala Asn Lys Ala Thr Leu Val Cys Leu Ile Ser Asp
       130                 135                 140
   Phe Tyr Pro Gly Ala Val Thr Val Ala Trp Lys Ala Asp Ser Ser Pro
   145                 150                 155                 160
   Val Lys Ala Gly Val Glu Thr Thr Thr Pro Ser Lys Gln Ser Asn Asn
                   165                 170                 175
   Lys Tyr Ala Ala Ser Ser Tyr Leu Ser Leu Thr Pro Glu Gln Trp Lys
               180                 185                 190
   Ser His Lys Ser Tyr Ser Cys Gln Val Thr His Glu Gly Ser Thr Val
           195                 200                 205
   Glu Lys Thr Val Ala Pro Ala Glu Cys Ser
       210                 215
<210> 8
   <211> 227
   <212> PRT
   <213> Homo sapiens
<400> 8
   Gln Val Gln Leu Gln Gln Ser Gly Ala Glu Val Lys Lys Pro Gly Ala
   1               5                   10                  15
   Ser Val Lys Val Ser Cys Lys Ala Ser Gly Tyr Thr Phe Thr Ser Tyr
               20                  25                  30
   Tyr Met His Trp Val Arg Gln Ala Pro Gly Gln Gly Leu Glu Trp Met
           35                  40                  45
   Gly Ile Ile Asn Pro Ser Gly Gly Ser Thr Ser Tyr Ala Gln Lys Phe
       50                  55                  60
   Gln Gly Arg Val Thr Met Thr Arg Asp Thr Ser Thr Ser Thr Val Tyr
   65                  70                  75                  80
   Met Glu Leu Ser Ser Leu Arg Ser Glu Asp Thr Ala Val Tyr Tyr Cys
                   85                  90                  95
   Ala Arg Asp Glu Gly Tyr Gly Asp Tyr Val Tyr Trp Gly Gln Gly Thr
               100                 105                 110
   Leu Val Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Ala Glu Pro Lys Ser Cys His His His
       210                 215                 220
   His His His
   225
<210> 9
   <211> 2133
   <212> DNA
   <213> Artificial Sequence
<220>
   <223> Artificial fusion protein: Homo sapiens + Escherichia coli
<400> 9
   caggtacagc tgcagcagtc aggggctgag gtgaagaagc ctggggcctc agtgaaggtt      60
   tcctgcaagg catctggata caccttcacc agctactata tgcactgggt gcgacaggcc     120
   cctggacaag ggcttgagtg gatgggaata atcaacccta gtggtggtag cacaagctac     180
   gcacagaagt tccagggcag agtcaccatg accagggaca cgtccacgag cacagtctac     240
   atggagctga gcagcctgag atctgaggac acggccgtgt attactgtgc gagagatgag     300
   ggctacggtg actacgtcta ctggggccag ggaaccctgg tcaccgtctc ctcactcgag     360
   ggtggtggtg gttctggggg cggaggatcc ggcgggggag ggtcagagct ccaggctgtg     420
   ctcactcagc cgtcctcagt gtctggggcc ccagggcaga gggtcaccat ctcctgcact     480
   ggaagcagct ccaacatcgg ggcaggttat gatgtacact ggtaccagca gcttccagga     540
   acagccccca aactcctcat ctatgggaac aacaatcggc cctcaggggt ccctgaccga     600
   ttctctggct ccaagtctgg aacctcagcc tccctggcca tcagtgggct ccagtctgag     660
   gacgaggctg attattactg tgcaacatgg gatgacagcc tgaatggtgt ggtatttggc     720
   ggagggacca aggtcaccgt cctaggtgcg gccgcacgta ctccggaaat gccggtgctg     780
   gaaaatcgtg ctgcacaggg cgatatcacg gcgcctggcg gtgctcgccg cttgacgggc     840
   gatcagaccg ctgctctgcg tgatagcctg agcgacaaac cggcgaaaaa catcattctg     900
   ctgattggcg atggtatggg cgacagcgag attaccgctg cgcgtaacta cgctgagggt     960
   gccggtggtt tctttaaagg tattgacgcg ctgccgctga cgggccaata cacccactac    1020
   gccctgaata agaaaacggg taagccggac tatgttaccg acagcgcggc atcggcaacc    1080
   gcgtggagca ccggcgtcaa aacctataac ggtgcgttgg gcgttgacat tcacgaaaag    1140
   gaccacccga ccatcctgga aatggcaaaa gcggctggtc tggcgacggg taacgttagc    1200
   accgcggaac tgcaagacgc gaccccggca gcactggtgg cacacgtcac cagccgtaaa     1260
   tgctacggtc cgagcgcaac cagcgaaaag tgcccgggta atgccctgga gaaaggtggc     1320
   aaaggttcca ttacggaaca actgctgaat gcgcgtgccg atgttacgct gggtggtggc     1380
   gccaagacgt ttgcggaaac ggcgaccgcg ggtgaatggc aaggcaaaac gttgcgcgaa     1440
   caagcgcagg cgcgtggcta ccaactggtt agcgacgcgg caagcctgaa cagcgttacc     1500
   gaggcgaacc agcaaaaacc gctgctgggt ctgttcgcgg acggtaacat gccggttcgt     1560
   tggctgggtc cgaaagccac gtaccacggt aatatcgata aaccggcggt tacgtgcact     1620
   ccgaatccgc aacgtaatga tagcgttccg accctggctc aaatgacgga caaagcaatt     1680
   gagctgctga gcaagaatga gaagggcttt ttcctgcaag ttgagggtgc gagcattgat     1740
   aaacaggatc atgcagcgaa cccgtgcggc cagattggtg aaaccgttga cctggacgaa     1800
   gcggtgcagc gtgcgctgga gttcgccaag aaagagggca ataccttggt catcgtcacc     1860
   gcggatcacg cacatgcgag ccagatcgtt gcaccggata cgaaggcacc gggcctgacc     1920
   caagcgctga acaccaaaga cggtgcggtt atggttatga gctacggcaa tagcgaagag     1980
   gatagccaag agcatacggg cagccaactg cgcattgcgg cctatggtcc gcacgcggcg     2040
   aatgtggtgg gtctgaccga tcaaaccgac ctgttttaca ccatgaaggc ggctctgggc     2100
   ctgaaaggcg gtggtcatca ccatcaccac cat                                  2133
<210> 10
   <211> 711
   <212> PRT
   <213> Artificial Sequence
<220>
   <223> Artificial fusion protein: Homo sapiens + Escherichia coli
<400> 10
   Gln Val Gln Leu Gln Gln Ser Gly Ala Glu Val Lys Lys Pro Gly Ala
   1               5                   10                  15
   Ser Val Lys Val Ser Cys Lys Ala Ser Gly Tyr Thr Phe Thr Ser Tyr
               20                  25                  30
   Tyr Met His Trp Val Arg Gln Ala Pro Gly Gln Gly Leu Glu Trp Met
           35                  40                  45
   Gly Ile Ile Asn Pro Ser Gly Gly Ser Thr Ser Tyr Ala Gln Lys Phe
       50                  55                  60
   Gln Gly Arg Val Thr Met Thr Arg Asp Thr Ser Thr Ser Thr Val Tyr
   65                  70                  75                  80
   Met Glu Leu Ser Ser Leu Arg Ser Glu Asp Thr Ala Val Tyr Tyr Cys
                   85                  90                  95
   Ala Arg Asp Glu Gly Tyr Gly Asp Tyr Val Tyr Trp Gly Gln Gly Thr
               100                 105                 110
   Leu Val Thr Val Ser Ser Leu Glu Gly Gly Gly Gly Ser Gly Gly Gly
           115                 120                 125
   Gly Ser Gly Gly Gly Gly Ser Glu Leu Gln Ala Val Leu Thr Gln Pro
       130                 135                 140
   Ser Ser Val Ser Gly Ala Pro Gly Gln Arg Val Thr Ile Ser Cys Thr
   145                 150                 155                 160
   Gly Ser Ser Ser Asn Ile Gly Ala Gly Tyr Asp Val His Trp Tyr Gln
                   165                 170                 175
   Gln Leu Pro Gly Thr Ala Pro Lys Leu Leu Ile Tyr Gly Asn Asn Asn
               180                 185                 190
   Arg Pro Ser Gly Val Pro Asp Arg Phe Ser Gly Ser Lys Ser Gly Thr
           195                 200                 205
   Ser Ala Ser Leu Ala Ile Ser Gly Leu Gln Ser Glu Asp Glu Ala Asp
       210                 215                 220
   Tyr Tyr Cys Ala Thr Trp Asp Asp Ser Leu Asn Gly Val Val Phe Gly
   225                 230                 235                 240
   Gly Gly Thr Lys Val Thr Val Leu Gly Ala Ala Ala Arg Thr Pro Glu
                   245                 250                 255
   Met Pro Val Leu Glu Asn Arg Ala Ala Gln Gly Asp Ile Thr Ala Pro
               260                 265                 270
   Gly Gly Ala Arg Arg Leu Thr Gly Asp Gln Thr Ala Ala Leu Arg Asp
           275                 280                 285
   Ser Leu Ser Asp Lys Pro Ala Lys Asn Ile Ile Leu Leu Ile Gly Asp
       290                 295                 300
   Gly Met Gly Asp Ser Glu Ile Thr Ala Ala Arg Asn Tyr Ala Glu Gly
   305                 310                 315                 320
   Ala Gly Gly Phe Phe Lys Gly Ile Asp Ala Leu Pro Leu Thr Gly Gln
                   325                 330                 335
   Tyr Thr His Tyr Ala Leu Asn Lys Lys Thr Gly Lys Pro Asp Tyr Val
               340                 345                 350
   Thr Asp Ser Ala Ala Ser Ala Thr Ala Trp Ser Thr Gly Val Lys Thr
           355                 360                 365
   Tyr Asn Gly Ala Leu Gly Val Asp Ile His Glu Lys Asp His Pro Thr
       370                 375                 380
   Ile Leu Glu Met Ala Lys Ala Ala Gly Leu Ala Thr Gly Asn Val Ser
   385                 390                 395                 400
   Thr Ala Glu Leu Gln Asp Ala Thr Pro Ala Ala Leu Val Ala His Val
                   405                 410                 415
   Thr Ser Arg Lys Cys Tyr Gly Pro Ser Ala Thr Ser Glu Lys Cys Pro
               420                 425                 430
   Gly Asn Ala Leu Glu Lys Gly Gly Lys Gly Ser Ile Thr Glu Gln Leu
           435                 440                 445
   Leu Asn Ala Arg Ala Asp Val Thr Leu Gly Gly Gly Ala Lys Thr Phe
       450                 455                 460
   Ala Glu Thr Ala Thr Ala Gly Glu Trp Gln Gly Lys Thr Leu Arg Glu
   465                 470                 475                 480
   Gln Ala Gln Ala Arg Gly Tyr Gln Leu Val Ser Asp Ala Ala Ser Leu
                   485                 490                 495
   Asn Ser Val Thr Glu Ala Asn Gln Gln Lys Pro Leu Leu Gly Leu Phe
               500                 505                 510
   Ala Asp Gly Asn Met Pro Val Arg Trp Leu Gly Pro Lys Ala Thr Tyr
           515                 520                 525
   His Gly Asn Ile Asp Lys Pro Ala Val Thr Cys Thr Pro Asn Pro Gln
       530                 535                 540
   Arg Asn Asp Ser Val Pro Thr Leu Ala Gln Met Thr Asp Lys Ala Ile
   545                 550                 555                 560
   Glu Leu Leu Ser Lys Asn Glu Lys Gly Phe Phe Leu Gln Val Glu Gly
                   565                 570                 575
   Ala Ser Ile Asp Lys Gln Asp His Ala Ala Asn Pro Cys Gly Gln Ile
               580                 585                 590
   Gly Glu Thr Val Asp Leu Asp Glu Ala Val Gln Arg Ala Leu Glu Phe
           595                 600                 605
   Ala Lys Lys Glu Gly Asn Thr Leu Val Ile Val Thr Ala Asp His Ala
       610                 615                 620
   His Ala Ser Gln Ile Val Ala Pro Asp Thr Lys Ala Pro Gly Leu Thr
   625                 630                 635                 640
   Gln Ala Leu Asn Thr Lys Asp Gly Ala Val Met Val Met Ser Tyr Gly
                   645                 650                 655
   Asn Ser Glu Glu Asp Ser Gln Glu His Thr Gly Ser Gln Leu Arg Ile
               660                 665                 670
   Ala Ala Tyr Gly Pro His Ala Ala Asn Val Val Gly Leu Thr Asp Gln
           675                 680                 685
   Thr Asp Leu Phe Tyr Thr Met Lys Ala Ala Leu Gly Leu Lys Gly Gly
       690                 695                 700
   Gly His His His His His His
   705                 710